(19) Europäisches Patentamt
European Patent Office
Office européen des brevets

(11) **EP 4 252 778 A2**

(12) **EUROPEAN PATENT APPLICATION**

(43) Date of publication:
04.10.2023 Bulletin 2023/40

(21) Application number: 23193127.0

(22) Date of filing: 28.12.2018

(51) International Patent Classification (IPC):
**A61K 47/36** (2006.01)

(52) Cooperative Patent Classification (CPC):
(C-Sets available)
**A61K 9/0014; A61K 31/015; A61K 31/05;
A61K 31/352; A61K 31/7048; A61K 36/185;
A61K 45/06; A61K 47/36; A61L 15/44;
A61L 26/0066; A61P 17/02; C07H 17/07;**
A61L 2300/216; A61L 2300/402     (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.04.2018 US 201862652650 P**

(62) Document number(s) of the earlier application(s) in
accordance with Art. 76 EPC:
**18913490.1 / 3 771 313**

(71) Applicant: **Vinsan Therapeutics Inc.
Woodbridge ON L4L 7B2 (CA)**

(72) Inventor: **MAIDA, Vincenzo
Woodbridge, Ontario, L4L 7B2 (CA)**

(74) Representative: **Porta & Consulenti Associati
S.p.A.
Via Vittoria Colonna, 4
20149 Milano (IT)**

Remarks:
This application was filed on 24-08-2023 as a
divisional application to the application mentioned
under INID code 62.

(54) **TOPICAL CANNABINOID FORMULATIONS AND INSTILLATES, KITS, AND METHODS FOR TREATING INTEGUMENTARY WOUNDS, AND USES THEREOF**

(57) Topical formulations comprising one or more cannabinoids, one or more terpenes, and one or more flavonoids; and methods and uses thereof for the treatment of an integumentary wound.

FIG. 2a

EP 4 252 778 A2

(52) Cooperative Patent Classification (CPC): (Cont.)

C-Sets
**A61K 31/015, A61K 2300/00;**
**A61K 31/05, A61K 2300/00;**
**A61K 31/352, A61K 2300/00;**
**A61K 31/7048, A61K 2300/00**

**Description**

**FIELD**

The present application relates generally to treatment of integumentary wounds (including chronic and acute wounds involving both cutaneous and mucous membranes), and in particular to topical formulations and instillates, kits, and methods for treating integumentary wounds, and uses thereof.

**BACKGROUND**

Wound healing normally progresses through highly organized and regulated sequence of events that are mediated by multiple cell lines and associated growth factors. Tissue damage, caused by either disease process or trauma, is followed by hemostasis. Thereafter, wound healing may be generally described to occur as three overlapping phases: the inflammatory, proliferative, and remodeling phases (Bielefeld et al. Cell. Mol. Life Sci. (2013) 70:2059-2081; and de Oliveira Gonzalez et al. An Bras Dermatol. 2016;91(5):614-20).

The inflammatory phase prepares the wound site for healing by immobilizing the wound and causing it to swell and become painful. The inflammatory phase also results in vasodilation and phagocytosis, which lead to release of histamines and serotonins.

The proliferative phase is characterized by the proliferation of epidermal cells at the wound edge, and by repair of the underlying dermal or mesenchymal layer. This is accompanied by neovascularization. This process usually occurs 2 days to 3 weeks following injury and results in granulation tissue at the wound site.

Granulation tissue formation occurs during the proliferative phase and involves the following mechanisms: an increase in fibroblastic proliferation; collagenous and elastic biosynthesis, which creates a three-dimensional extracellular network of connective tissue; and the production of chemotactic factors and IFN-beta by fibroblasts, (de Oliveira Gonzalez et al.) Healthy granulation tissue is granular and uneven in texture; it does not bleed easily and is pink / red in colour.

In the final remodeling phase, remodeling of dermal tissue to produce greater tensile strength whereby new collagen is formed is the main aim of this phase. The principle cell type involved is the fibroblast. Collagen molecules begin to form whereby they undergo further modification and molecules begin to form in a characteristic triple helical structure. Together, these changes result in the contraction of the wound and the formation of acellular scar tissue.

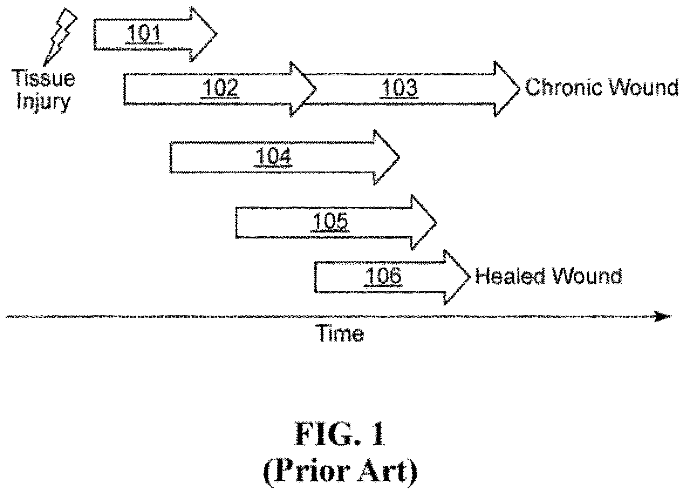

FIG. 1 illustrates an exemplary normal wound healing sequence: upon tissue injury, hemostatsis 101 occurs which may involve epinephrine, platelets, and transforming growth factor beta (TGF-β); inflammation 102 may follow hemostatsis 101 and involve neutrophils, macrophages, reactive oxygen species, matrix metalloproteinases (MMPs), interleukins (IL), tumor necrosis factors (TNF), vascular endothelial growth factor (VEGF), TGF-β, and platelet-derived growth factor (PDGF); inflammation 102 may become prolonged inflammation 103 that may lead to a chronic wound; granulation and angiogenesis 104 may follow inflammation 102 and involve fibroblasts, macrophages, endothelial cells, MMPs, IL, TNF, VEGF, TGF-β, PDGF, and keratinocyte growth factor (KGF); re-epithelialization 105 may follow granulation and angiogenesis 104 and involve keratinocytes, endothelial cells, epidermal growth factor, KGF, and MMPs; and tissue remodeling 106 may follow re-epithelialization 105 and involve fibroblasts, collagen fiber cross-linking, TGF-β, and MMPs, which may lead to a healed wound.

With numerous disease processes, the cascade of events involved in wound healing can be affected, resulting in chronic, non-healing wounds. This could be due to a complex combination of local and systemic factors. The pathophysiology of the "stalled" or "arrested" healing process may be characterized by a state of heightened and prolonged inflammation.

In an exemplary chronic wound cycle, prolonged inflammation stimulates macrophages and neutrophils to wound where pro-inflammatory cytokines such as TNFα and IL-1β are released; release of these cytokines lead to increased expression of MMPs and decreased expression of tissue inhibitor of metalloproteinase, which contribute to degradation of extracellular matrix (resulting in impaired cell migration and connective tissue deposition) and growth factors, thereby reinforcing prolonged inflammation. Effects of this exemplary chronic wound cycle may include delayed healing, repeated trauma, local tissue ischemia, necrotic tissue, heavy bacterial burden and tissue breakdown.

Chronic wounds, wounds that fail to heal in an orderly and timely fashion, can generate untold suffering, reduced quality of life, lost productivity, loss of limbs, and reduced life expectancy, while consuming ever increasing proportions of global healthcare budgets. The United States of America spends in excess of 90 billion dollars annually on overall wound management and this is growing faster than any other area within healthcare, approaching 8% per annum.

While there are topical wound therapies and dressings available based on anecdotal experience, few have published or prospective data to support their effectiveness in promoting wound healing. There are also a number of advanced therapies, such as negative pressure wound therapy and hyperbaric oxygen therapy, but these advanced therapies often require special equipment/devices or surgical procedures. For example, negative pressure wound therapy requires a regulated vacuum dressing and hyperbaric oxygen therapy requires a hyperbaric oxygen chamber.

The overall treatment of wounds is recognized as one of the most dismally managed domains within global healthcare. Therefore, there exists a need for the development of wound therapies, dressings, and protocols that are effective in promoting wound healing and easy to be administered to patients.

## SUMMARY

In one aspect, there is provided a topical instillate comprising:

(a) 0.1 mg/ml to 40 mg/ml of one or more cannabinoids;

(b) 25 mg/ml to 1000 mg/ml of one or more terpenes;

(c) 10 mg/ml to 500 mg/ml of one or more flavonoids; and

(d) a liquid carrier selected for instillation of the instillate onto an integumentary wound.

In an embodiment of the topical instillate as described herein, the one or more cannabinoids comprise at least one of cannabidiol and tetrahydrocannabinol.

In an embodiment of the topical instillate as described herein, the one or more terpenes comprise beta-caryophyllene, and the concentration of beta-caryophyllene is 50 mg/ml to 500 mg/ml.

In an embodiment of the topical instillate as described herein, the one or more flavonoids comprise at least one of diosmin and quercetin.

In another aspect, there is provided a topical formulation for direct application to an integumentary wound, comprising:

(a) 0.1 mg/ml to 40 mg/ml of one or more cannabinoids;

(b) 25 mg/ml to 1000 mg/ml of one or more terpenes; and

(c) 10 mg/ml to 500 mg/ml of one or more flavonoids.

In an embodiment of the topical formulation as described herein, the one or more cannabinoids comprise at least one of cannabidiol and tetrahydrocannabinol.

In an embodiment of the topical formulation as described herein, the one or more terpenes comprise beta-caryophyllene, and the concentration of beta-caryophyllene is 50 mg/ml to 500 mg/ml.

In an embodiment of the topical formulation as described herein, the one or more flavonoids comprise at least one of diosmin, quercetin, and hesperidin.

In an embodiment of the topical formulation as described herein, the topical formulation further comprises a liquid carrier selected for instillation of the topical formulation onto an integumentary wound.

In another aspect, there is provided a wound dressing comprising a contact layer, and the topical instillate as described herein or the topical formulation as described herein integrated with the contact layer.

In another aspect, there is provided a method comprising instilling a topical formulation onto an integumentary wound of a subject, wherein the topical formulation comprises one or more cannabinoids; one or more terpenes; and one or more flavonoids.

In an embodiment of the method, the instilling comprises dropping, spraying, diffusing, dispersing, squirting, or spreading the topical formulation.

In an embodiment of the method, the integumentary wound is acute, or is chronic, stalled, recalcitrant, or a combination thereof.

In an embodiment of the method, the method further comprises instilling the topical formulation onto a periwound area around the integumentary wound.

In another aspect, there is provided use of a topical formulation for the treatment of an integumentary wound of a subject, wherein the topical formulation comprises one or more cannabinoids; one or more terpenes; and one or more flavonoids.

In an embodiment of the use as described herein, the topical formulation is used for instillation onto the integumentary wound.

In an embodiment of the use as described herein, the integumentary wound is acute, or is chronic, stalled, recalcitrant, or a combination thereof.

In an embodiment of the use as described herein, the topical formulation is further used for instillation onto a periwound area around the integumentary wound.

In another aspect, there is provided a kit comprising (i) a container containing the topical instillate as described herein or the topical formulation as described herein, and instructions for instilling the topical instillate or topical formulation onto an integumentary wound; or (ii) a plurality of containers containing materials for forming the topical instillate or topical formulation, and instructions for preparing the topical instillate or topical formulation from the materials in the containers and for instilling the topical instillate or topical formulation onto the integumentary wound.

Other aspects, features, and embodiments of the present disclosure will become apparent to those of ordinary skill in the art upon review of the following description of specific embodiments in conjunction with the accompanying figures.

## BRIEF DESCRIPTION OF THE FIGURES

FIG. 1 is a diagram showing an exemplary normal wound healing sequence.

FIG. 2a is a schematic section view of a portion of normal tissues with intact skin.

FIG. 2b is a schematic section view of wounded tissues with an exposed wound bed.

FIGS. 2c-2g are schematic section views of the wound of FIG. 2b during treatment illustrating a treatment process according embodiments disclosed herein.

FIG. 3a shows representative analysis performed on day 15 in Example 2.

FIG. 3b shows representative analysis performed on day 41 in Example 2.

FIG. 3c shows representative analysis performed on day 87 in Example 2.

FIG. 4 shows the trend of wound healing as represented by the granulation and epithelial tissue density inside a wound area. Accounting of epithelial tissue started on approximately day 30 when significant epithelium growth started.

FIG. 5a shows wound size as measured by longest length.

FIG. 5b shows wound size as measured by widest width.

FIG. 5c shows wound size as measured by product of longest length and widest width as an upper-bound estimate of total wound area.

FIG. 6 is a schematic block diagram illustrating an exemplary kit according to an embodiment of the present disclosure.

## DETAILED DESCRIPTION

It has been surprisingly discovered by the present inventor that treating an integumentary wound with a selected combination of a cannabinoid, a terpene and a flavonoid applied directly onto the wound bed can provide healing effects, such as stimulating or facilitating granulation tissue growth and promoting epithelialization of the integumentary wound.

As used herein, "integument" refers to the outer protective layer(s), both cutaneous membrane and mucous membrane, of a living being and the term "integumentary wound" refers to a breakdown and loss of at least a portion of the integument including the outermost sublayer(s) of the integument, namely the epidermis, and optionally destruction of deeper tissues such as the dermis, fat, fascial connective tissues, and often muscle and bone. An integumentary wound may include a wound commonly referred to as an open wound, where an injured body area exposes the dermal layer of skin or tissue(s) and structure(s) beneath the dermal layer (such as fat, muscle, fascia, and bone) of skin to air. As can be appreciated by those skilled in the art, the integument has two main layers: (i) the outer layer, referred to as the epidermis, which functions as a barrier to the external environment, and (ii) the inner layer, referred to as the dermis, which is composed of connective tissue and provides the skin with some of its mechanical properties.

In an embodiment, treatment of an integumentary wound includes topically delivering the selected cannabinoid(s), terpene(s) and flavonoid(s) to the integumentary wound as instillates.

An embodiment of the present disclosure thus provides a topical formulation comprising a selected composition of cannabinoids, terpenes and flavonoids, at concentrations within specific respective ranges, formulated for direct application onto an integumentary wound.

For example, in a particular embodiment, the formulation may include a topical instillate, which includes: (a) 5 mg/ml to 30 mg/ml of cannabidiol and 2 mg/ml to 10 mg/ml of tetrahydrocannabinol; (b) 30 mg/ml to 60 mg/ml of beta-caryophyllene and 10 mg/ml to 30 mg/ml of linalool; (c) 10 mg/ml to 30 mg/ml of diosmin and 10 mg/ml to 30 mg/ml of quercetin; and (d) aloe vera gel and optionally hyaluronic acid gel.

In another particular embodiment, the formulation may include a topical instillate, which includes: (a) 0.1 mg/ml to 20 mg/ml of cannabidiol and 0 mg/ml to 5 mg/ml of tetrahydrocannabinol; (b) 50 mg/ml to 500 mg/ml of beta-caryophyllene and 10 mg/ml to 150 mg/ml of linalool; (c) 0 mg/ml to 50 mg/ml of diosmin and 10 mg/ml to 50 mg/ml of quercetin; and (d) aloe vera gel and optionally hyaluronic acid gel.

In another particular embodiment, the formulation may include a topical instillate, which includes: (a) 2.3 mg/ml of cannabidiol and 1.0 mg/ml of tetrahydrocannabinol; (b) 81.5 mg/ml of beta-caryophyllene and 28.4 mg/ml of linalool; (c) 16.7 mg/ml of micronized diosmin and 16.7 mg/ml of micronized quercetin; and (d) aloe vera gel and hyaluronic acid gel.

In another particular embodiment, the formulation may include a topical instillate, which includes: (a) 2.6 mg/ml of cannabidiol; (b) 118 mg/ml of beta-caryophyllene; (c) 19.6 mg/ml of micronized diosmin, 21.7 mg/ml of micronized quercetin, and 2.2 mg/ml of hersperidin; and (d) aloe vera gel and hyaluronic acid gel.

Unless indicated otherwise, the stated concentrations in the present disclosure are based on the total volume of the formulation and the dry weights of respective active agents.

The formulation may include a solution or a colloid, and is formulated for direct application to the wound bed of an integumentary wound via instillation, such as by dropping, spraying, diffusing, dispersing, squirting, or spreading the formulation onto the integumentary wound bed, to promote wound healing.

Further embodiments of the present disclosure relate to methods of treating integumentary wounds. In a particular method, the method includes directly applying a topical formulation comprising a cannabinoid, a terpene, and a flavonoid onto an integumentary wound.

Still further embodiments of the present disclosure relate to uses of selected topical formulations disclosed herein for the treatment of an integumentary wound.

Selected topical formulations disclosed herein may also have one or more other beneficial effects such as management of pain (e.g., baseline pain and breakthrough pain), analgesic effects, anti-inflammatory effects, anti-pruritic effects, opioid-sparing effects, anti-microbial activity or the like.

Topical Formulations

The term "topical formulation" is generally understood to mean a mixture of substances that is suitable for application to a particular place on or in the body. A topical formulation may be a solution in which one or more solutes are uniformly distributed within a solvent, or a colloid in which one substance is not dissolved in, but suspended throughout, another substance. A topical formulation may exist in any phase or a combination of phases. Within the context of the present disclosure, suitable forms of a topical formulation for application to a cutaneous wound may include solution, lotion, cream, ointment, gel, emulsion, liposome, foam, powder, impregnated gauze sheet, tulle, vapor and paste, and suitable forms of a topical formulation for application to a mucous wound may include aerosolized spray for nasal and oral applications and suppository for rectal and vaginal applications.

In an embodiment, a topical formulation may include one or more cannabinoids, one or more terpenes, one or more flavonoids, and a liquid carrier selected for instillation of the topical formulation onto an integumentary wound.

The term "cannabinoid" is generally understood to include any chemical compound that acts upon a cannabinoid receptor. Examples of cannabinoids include cannabidiol (CBD), cannabinol (CBN), cannabigerol (CBG), cannabichromene (CBC), tetrahydrocannabivarin (THCV), cannabichromanon (CBCN), cannabielsoin (CBE), cannbifuran (CBF), tetrahydrocannabinol (THC), cannabinodiol (CBDL), cannabicyclol (CBL), cannabitriol (CBT), cannabivarin (CBV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinodiol (CBND), cannabinol propyl variant (CBNV), cannabitriol (CBO), tetrahydrocannabinolic acid (THCA), tetrahydrocannabivarinic acid (THCVA), and derivatives thereof. Further examples of cannabinoids are discussed in PCT Patent Application Pub. No. WO2017/190249 and US Patent Application Pub. No. US2014/0271940.

A cannabinoid may be in an acid form or a non-acid form, the latter also being referred to as the decarboxylated form since the non-acid form can be generated by decarboxylating the acid form. Within the context of the present disclosure, where reference is made to a particular cannabinoid, the cannabinoid can be in its acid or non-acid form, or be a mixture of both acid and non-acid forms.

In some embodiments, a topical formulation provided herein may include cannabidiol (CBD). CBD is not psychoactive, and is expected to relieve convulsion, inflammation, anxiety, and nausea.

The terms "cannabidiol," "CBD," or "cannabidiols" are generally understood to refer to one or more of the following compounds, and, unless a particular other stereoisomer or stereoisomers are specified, includes the compound "$\Delta^2$-cannabidiol." These compounds are: (1) $\Delta^5$-cannabidiol (2-(6-isopropenyl-3-methyl-5-cyclohexen-1-yl)-5-pentyl-1,3-benzenediol); (2) $\Delta^4$-cannabidiol (2-(6-isopropenyl-3-methyl-4-cyclohexen-1-yl)-5-pentyl-1,3-benzenediol); (3) $\Delta^3$-cannabidiol (2-(6-isopropenyl-3-methyl-3-cyclohexen-1-yl)-5-pentyl-1,3-benzenediol); (4) $\Delta^{3,7}$- cannabidiol (2-(6-isopropenyl-3-methylenecyclohex-1-yl)-5-pentyl-1,3-benzenediol); (5) $\Delta^2$- cannabidiol (2-((6-isopropenyl-3-methyl-2-cyclohexen-1-yl)-5-pentyl-1,3-benzenediol); (6) $\Delta^1$- cannabidiol (2-(6-isopropenyl-3-methyl-1-cyclohexen-1-yl)-5-pentyl-1,3-benzenediol); and (7) $\Delta^6$-cannabidiol (2-(6-isopropenyl-3-methyl-6-cyclohexen-1-yl)-5-pentyl-1,3-benzenediol).

These compounds have one or more chiral centers and two or more stereoisomers as stated below: (1) $\Delta^5$-cannabidiol has 2 chiral centers and 4 stereoisomers; (2) $\Delta^4$-cannabidiol has 3 chiral centers and 8 stereoisomers; (3) $\Delta^3$-cannabidiol has 2 chiral centers and 4 stereoisomers; (4) $\Delta^{3,7}$- cannabidiol has 2 chiral centers and 4 isomers; (5) $\Delta^2$-cannabidiol has 2 chiral centers and 4 stereoisomers; (6) $\Delta^1$- cannabidiol has 2 chiral centers and 4 stereoisomers; and (7) $\Delta^6$-cannabidiol has 1 chiral center and 2 stereoisomers.

In some embodiments, a topical formulation provided herein may include $\Delta^2$-cannabidiol.

Unless specifically stated, a reference to "cannabidiol," "CBD," or "cannabidiols" or to any of specific cannabidiol compounds (1)-(7) as referred to above includes all possible stereoisomers of all compounds included by the reference. For example, "$\Delta^2$-cannabidiol" may be a mixture of the $\Delta^2$-cannabidiol stereoisomers that are present in a plant, or an extract thereof, such as Cannabis sativa, Cannabis indica, or another plant of the Cannabis genus;"$\Delta^2$-cannabidiol" may be a mixture of the $\Delta^2$-cannabidiol stereoisomers that are present in a plant, or an extract thereof, such as Cannabis sativa, Cannabis indica, or another plant of the Cannabis genus, wherein said mixture of stereoisomers is at, or at about, the naturally occurring ratio of isomers; and "$\Delta^2$-cannabidiol" may be a single stereoisomer.

In some embodiments, a topical formulation provided herein may comprise one or more cannabinoids, such as cannabinol, cannabigerol, cannabichromene, and tetrahydrocannabivarin, in addition to CBD. The combination of CBD and CBN may be particularly useful for managing burn pain.

In some embodiments, a topical formulation provided herein may also include THC. In other embodiments, a topical formulation provided herein may not contain THC. THC is only psychoactive in its decarboxylated state. The carboxylic acid form (THCa) is non-psychoactive. Delta-9-tetrahydrocannabinol ($\Delta$9-THC) and delta-8-tetrahydrocannabinol ($\Delta$8-THC) produce the effects associated with cannabis by binding to the CB1 cannabinoid receptors in the brain. THC is expected to ease moderate pain (analgesic) and to be neuroprotective, while also offering the potential to reduce neuroinflammation and to stimulate neurogenesis. In some embodiments, THC may be substituted entirely by THCV.

In some embodiments, a topical formulation provided herein may include 0.1 mg/ml to 40 mg/ml of cannabinoid(s). For example, a topical formulation provided herein may comprise 0.1 mg/ml to 30 mg/ml, 0.5 mg/ml to 30 mg/ml, 1 mg/ml to 30 mg/ml, 0.1 mg/ml to 25 mg/ml, 0.5 mg/ml to 25 mg/ml, 1 mg/ml to 25 mg/ml, 0.1 mg/ml to 20 mg/ml, 0.5 mg/ml to 20 mg/ml, 1 mg/ml to 20 mg/ml, 0.1 mg/ml to 15 mg/ml, 0.5 mg/ml to 15 mg/ml, 1 mg/ml to 15 mg/ml, 0.1 mg/ml to 10 mg/ml, 0.5 mg/ml to 10 mg/ml, 1 mg/ml to 10 mg/ml, 0.1 mg/ml to 5 mg/ml, 0.5 mg/ml to 5 mg/ml, 1 mg/ml to 5 mg/ml, 0.1 mg/ml to 2 mg/ml, 0.5 mg/ml to 2 mg/ml, 1 mg/ml to 2 mg/ml, 2 mg/ml to 40 mg/ml, 2 mg/ml to 30 mg/ml, 2 mg/ml to 25 mg/ml, 2 mg/ml to 20 mg/ml, 2 mg/ml to 15 mg/ml, 2 mg/ml to 10 mg/ml, 2 mg/ml to 5 mg/ml, 5 mg/ml to 40 mg/ml, 5 mg/ml to 30 mg/ml, 5 mg/ml to 25 mg/ml, 5 mg/ml to 20 mg/ml, 5 mg/ml to 15 mg/ml, 5 mg/ml to 10 mg/ml, 10 mg/ml to 40 mg/ml, 10 mg/ml to 30 mg/ml, 10 mg/ml to 25 mg/ml, 10 mg/ml to 20 mg/ml, 10 mg/ml to 15 mg/ml, 15 mg/ml to 40 mg/ml, 15 mg/ml to 30 mg/ml, 15 mg/ml to 25 mg/ml, 15 mg/ml to 20 mg/ml, 20 mg/ml to 40 mg/ml, 20 mg/ml to 30 mg/ml, 20 mg/ml to 25 mg/ml, 25 mg/ml to 40 mg/ml, 25 mg/ml to 30 mg/ml, or 30 mg/ml to 40 mg/ml of cannabinoid(s).

In some embodiments, a topical formulation provided herein may comprise at least 0.1 mg/ml, 0.5 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, or 39 mg/ml of cannabinoid(s).

In some embodiments, a topical formulation provided herein may comprise 0.1 mg/ml, 0.5 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml or 40 mg/ml of cannabinoid(s).

In some embodiments, the concentrations of cannabinoids in a topical formulation provided herein may be adjusted depending on the phase of wound healing. For example, during the inflammatory phase, a higher level of a mixture of THC and CBD (e.g., 5 mg/ml to 20 mg/ml of cannabidiol and 2 mg/ml to 10 mg/ml of tetrahydrocannabinol) can be beneficial since wound pain is most intense during this phase and higher levels of THC and CBD can help to manage the pain. In comparison, during the re-epithelial and remodeling phases, a reduced concentration of THC (e.g., 0 mg/ml to 5 mg/ml) may be desirable since preclinical studies suggest that THC may inhibit keratinocyte differentiation, while the CBD concentration may remain relatively high (e.g., 0.1 mg/ml to 20 mg/ml).

The term "terpene" is generally understood to include any organic compound derived biosynthetically from units of isoprene, and the term "terpenoid" generally refers to a chemically modified terpene (e.g., by oxidation). As used herein, terpenes include terpenoids. Terpenes may be classified in various ways, such as by their sizes. For example, suitable terpenes may include monoterpenes, sesquiterpenes,or triterpenes. At least some terpenes are expected to interact with, and potentiate the activity of, cannabinoids.

Examples of terpenes known to be extractable from cannabis include aromadendrene, bergamottin, bergamotol, bisabolene, borneol, 4-3-carene, beta-caryophyllene, cineole/eucalyptol, p-cymene, dihydroj asmone, elemene, farnesene, fenchol, geranylacetate, guaiol, humulene, isopulegol, limonene, linalool, menthone, menthol, menthofuran, myrcene, nerylacetate, neomenthylacetate, ocimene, perillylalcohol, phellandrene, pinene, pulegone, sabinene, terpinene, terpineol, terpineol-4-ol, terpinolene, and derivatives thereof.

Additional examples of terpenes include nerolidol, phytol, geraniol, alpha-bisabolol, thymol, genipin, astragaloside, asiaticoside, camphene, beta-amyrin, thujone, citronellol, 1,8-cineole, cycloartenol, and derivatives thereof. Further examples of terpenes are discussed in US Patent Application Pub. No. US2016/0250270.

In some embodiments, a topical formulation provided herein may include at least one of beta-caryophyllene, linalool, thymol, alpha-bisabolol, myrcene, limonene, and pinene. In some embodiments, a topical formulation provided herein may comprise beta-caryophyllene and a monoterpene such as linalool, thymol, alpha-bisabolol, alpha-terpineol and genipin or a triterpene such as astragaloside and asiaticoside. In some embodiments, a topical formulation provided herein may comprise beta-caryophyllene, linalool, or both.

Cannabinoid oils available on the market often contain trace amounts of various terpenes. In some embodiments, a topical formulation provided herein may have a total concentration of terpene(s) that is higher than the total concentration of terpenes found in commercially available cannabinoid oils.

For example, in some embodiments, a topical formulation provided herein may include 25 mg/ml to 1000 mg/ml of terpene(s). More particularly, the total terpene concentration in a topical formulation provided herein may be 25 mg/ml to 1000 mg/ml, 25 mg/ml to 500 mg/ml, 25 mg/ml to 400 mg/ml, 25 mg/ml to 300 mg/ml, 25 mg/ml to 250 mg/ml, 25 mg/ml to 200 mg/ml, 25 mg/ml to 180 mg/ml, 25 mg/ml to 160 mg/ml, 25 mg/ml to 140 mg/ml, 25 mg/ml to 120 mg/ml, 25 mg/ml to 100 mg/ml, 25 mg/ml to 80 mg/ml, 25 mg/ml to 60 mg/ml, 25 mg/ml to 40 mg/ml, 40 mg/ml to 1000 mg/ml, 40 mg/ml to 500 mg/ml, 40 mg/ml to 400 mg/ml, 40 mg/ml to 300 mg/ml, 40 mg/ml to 250 mg/ml, 40 mg/ml to 200 mg/ml, 40 mg/ml to 180 mg/ml, 40 mg/ml to 160 mg/ml, 40 mg/ml to 140 mg/ml, 40 mg/ml to 120 mg/ml, 40 mg/ml to 100 mg/ml, 40 mg/ml to 90 mg/ml, 40 mg/ml to 80 mg/ml, 40 mg/ml to 60 mg/ml, 60 mg/ml to 1000 mg/ml, 60 mg/ml to 500 mg/ml, 60 mg/ml to 400 mg/ml, 60 mg/ml to 300 mg/ml, 60 mg/ml to 250 mg/ml, 60 mg/ml to 200 mg/ml, 60 mg/ml to 180 mg/ml, 60 mg/ml to 160 mg/ml, 60 mg/ml to 140 mg/ml, 60 mg/ml to 120 mg/ml, 60 mg/ml to 100 mg/ml, 60 mg/ml to 80 mg/ml, 80 mg/ml to 1000 mg/ml, 80 mg/ml to 500 mg/ml, 80 mg/ml to 400 mg/ml, 80 mg/ml to 300 mg/ml, 80 mg/ml to 250 mg/ml, 80 mg/ml to 200 mg/ml, 80 mg/ml to 180 mg/ml, 80 mg/ml to 160 mg/ml, 80 mg/ml to 140 mg/ml, 80 mg/ml to 120 mg/ml, 80 mg/ml to 100 mg/ml, 100 mg/ml to 1000 mg/ml, 100 mg/ml to 500 mg/ml, 100 mg/ml to 400 mg/ml, 100 mg/ml to 300 mg/ml, 100 mg/ml to 250 mg/ml, 100 mg/ml to 200 mg/ml, 100 mg/ml to 180 mg/ml, 100 mg/ml to 160 mg/ml, 100 mg/ml to 140 mg/ml, 100 mg/ml to 120 mg/ml, 120 mg/ml to 1000 mg/ml, 120 mg/ml to 500 mg/ml, 120 mg/ml to 400 mg/ml, 120 mg/ml to 300 mg/ml, 120 mg/ml to 250 mg/ml, 120 mg/ml to 200 mg/ml, 120 mg/ml to 180 mg/ml, 120 mg/ml to 160 mg/ml, 120 mg/ml to 140 mg/ml, 140 mg/ml to 1000 mg/ml, 140 mg/ml to 500 mg/ml, 140 mg/ml to 400 mg/ml, 140 mg/ml to 300 mg/ml, 140 mg/ml to 250 mg/ml, 140 mg/ml to 200 mg/ml, 140 mg/ml to 180 mg/ml, 140 mg/ml to 160 mg/ml, 160 mg/ml to 1000 mg/ml, 160 mg/ml to 500 mg/ml, 160 mg/ml to 400 mg/ml, 160 mg/ml to 300 mg/ml, 160 mg/ml to 250 mg/ml, 160 mg/ml to 200 mg/ml, 160 mg/ml to 180 mg/ml, 180 mg/ml to 1000 mg/ml, 180 mg/ml to 500 mg/ml, 180 mg/ml to 400 mg/ml, 180 mg/ml to 300 mg/ml, 180 mg/ml to 250 mg/ml, 180 mg/ml to 200 mg/ml, 200 mg/ml to 1000 mg/ml, 200 mg/ml to 500 mg/ml, 200 mg/ml to 400 mg/ml, 200 mg/ml to 300 mg/ml, or 200 mg/ml to 250 mg/ml.

In some embodiments, the total terpene concentration in a topical formulation provided herein may be at least 25 mg/ml, 30 mg/ml, 35 mg/ml, 40 mg/ml, 45 mg/ml, 50 mg/ml, 55 mg/ml, 60 mg/ml, 65 mg/ml, 70 mg/ml, 75 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 300 mg/ml, 400 mg/ml, 500 mg/ml, 600 mg/ml, 700 mg/ml, 800 mg/ml, or 900 mg/ml.

In some embodiments, the total terpene concentration in a topical formulation provided herein may be 25 mg/ml, 30 mg/ml, 35 mg/ml, 40 mg/ml, 45 mg/ml, 50 mg/ml, 55 mg/ml, 60 mg/ml, 65 mg/ml, 70 mg/ml, 75 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 300 mg/ml, 400 mg/ml, 500 mg/ml, 600 mg/ml, 700 mg/ml, 800 mg/ml, or 900 mg/ml.

In some embodiments, the concentrations of terpenes in a topical formulation provided herein may be adjusted depending on the phase of wound healing. For example, it has been discovered that a high level of beta-caryophyllene (e.g., 50 mg/ml to 500 mg/ml) may be desirable during the re-epithelialization and remodeling phases as beta-caryophyllene is a strong agonist for CB2 receptors of the endocannabinoid system.

The term "flavonoid" is generally understood to include any secondary plant metabolite that has a general 15-carbon skeleton structure which consists of two phenyl rings and a heterocyclic ring. Flavonoids are generally classified into subclasses by the state of oxidation and the substitution pattern at the C2-C3 unit, including flavanones, flavonols, flavones, anthocyanidins, chalcones, dihydrochalcones, aurones, flavanols, dihydroflavanols, proanthocyanidins (flavan-3,4-diols), isoflavones and neoflavones. Specific examples of flavonoids include kaempferol (3,4',5,7-tetrahydroxyflavone), apigenin (4',5,7-trihydroxyflavone), chrysin, diosmin, hesperidin, luteolin, rutin, and quercetin.

In some embodiments, a topical formulation provided herein may include quercetin. In some embodiments, a topical formulation provided herein may include diosmin, quercetin, hesperidin, or a combination thereof. In some embodiments, a topical formulation provided herein may include quercetin, kaempferol, apigenin, or a combination thereof. Each or both of diosmin and quercetin may be micronized, and optionally in the form of dry powders.

Cannabinoid oils available on the market often contain trace amounts of various flavonoids. In some embodiments, a topical formulation provided herein may have a total flavonoid concentration that is higher than the total concentration of flavonoids found in commercially available cannabinoid oils.

In some embodiments, the total flavonoid concentration in a topical formulation provided herein may be 10 mg/ml to 500 mg/ml. For example, the total flavonoid concentration may be 10 mg/ml to 400 mg/ml, 10 mg/ml to 300 mg/ml, 10 mg/ml to 200 mg/ml, 10 mg/ml to 150 mg/ml, 10 mg/ml to 100 mg/ml, 10 mg/ml to 90 mg/ml, 10 mg/ml to 80 mg/ml, 10 mg/ml to 70 mg/ml, 10 mg/ml to 60 mg/ml, 10 mg/ml to 50 mg/ml, 10 mg/ml to 40 mg/ml, 10 mg/ml to 30 mg/ml, 10 mg/ml to 25 mg/ml, 10 mg/ml to 20 mg/ml, 10 mg/ml to 15 mg/ml, 15 mg/ml to 500 mg/ml, 15 mg/ml to 400 mg/ml, 15 mg/ml to 300 mg/ml, 15 mg/ml to 200 mg/ml, 15 mg/ml to 150 mg/ml, 15 mg/ml to 100 mg/ml, 15 mg/ml to 90 mg/ml, 15 mg/ml to 80 mg/ml, 15 mg/ml to 70 mg/ml, 15 mg/ml to 60 mg/ml, 15 mg/ml to 50 mg/ml, 15 mg/ml to 40 mg/ml, 15 mg/ml to 30 mg/ml, 15 mg/ml to 25 mg/ml, 15 mg/ml to 20 mg/ml, 20 mg/ml to 500 mg/ml, 20 mg/ml to 400 mg/ml, 20 mg/ml to 300 mg/ml, 20 mg/ml to 200 mg/ml, 20 mg/ml to 150 mg/ml, 20 mg/ml to 100 mg/ml, 20 mg/ml to 90 mg/ml, 20 mg/ml to 80 mg/ml, 20 mg/ml to 70 mg/ml, 20 mg/ml to 60 mg/ml, 20 mg/ml to 50 mg/ml, 20 mg/ml to 40 mg/ml, 20 mg/ml to 30 mg/ml, 40 mg/ml to 500 mg/ml, 40 mg/ml to 400 mg/ml, 40 mg/ml to 300 mg/ml, 40 mg/ml to 200 mg/ml, 40 mg/ml to 150 mg/ml, 40 mg/ml to 100 mg/ml, 40 mg/ml to 90 mg/ml, 40 mg/ml to 80 mg/ml, 40 mg/ml to 70 mg/ml, 40 mg/ml to 60 mg/ml, 40 mg/ml to 50 mg/ml, 50 mg/ml to 500 mg/ml, 50 mg/ml to 400 mg/ml, 50 mg/ml to 300 mg/ml, 50 mg/ml to 200 mg/ml, 50 mg/ml to 150 mg/ml, 50 mg/ml to 100 mg/ml, 50 mg/ml to 90 mg/ml, 50 mg/ml to 80 mg/ml, 50 mg/ml to 70 mg/ml, 50 mg/ml to 60 mg/ml, 60 mg/ml to 500 mg/ml, 60 mg/ml to 400 mg/ml, 60 mg/ml to 300 mg/ml, 60 mg/ml to 200 mg/ml, 60 mg/ml to 150 mg/ml, 60 mg/ml to 100 mg/ml, 60 mg/ml to 90 mg/ml, 60 mg/ml to 80 mg/ml, 60 mg/ml to 70 mg/ml, 80 mg/ml to 500 mg/ml, 80 mg/ml to 400 mg/ml, 80 mg/ml to 300 mg/ml, 80 mg/ml to 200 mg/ml, 80 mg/ml to 150 mg/ml, 80 mg/ml to 100 mg/ml, 80 mg/ml to 90 mg/ml, 90 mg/ml to 500 mg/ml, 90 mg/ml to 400 mg/ml, 90 mg/ml to 300 mg/ml, 90 mg/ml to 200 mg/ml, 90 mg/ml to 150 mg/ml, 90 mg/ml to 100 mg/ml, 100 mg/ml to 500 mg/ml, 100 mg/ml to 400 mg/ml, 100 mg/ml to 300 mg/ml, 100 mg/ml to 200 mg/ml, 100 mg/ml to 150 mg/ml, 150 mg/ml to 500 mg/ml, 150 mg/ml to 400 mg/ml, 150 mg/ml to 300 mg/ml, 150 mg/ml to 200 mg/ml, 200 mg/ml to 500 mg/ml, 200 mg/ml to 400 mg/ml, 200 mg/ml to 300 mg/ml, 300 mg/ml to 500 mg/ml, 300 mg/ml to 400 mg/ml, or 400 mg/ml to 500 mg/ml.

In some embodiments, the total flavonoid concentration in a topical formulation provided herein may be at least 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 30 mg/ml, 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 250 mg/ml, 300 mg/ml, 350 mg/ml, 400 mg/ml, or 450 mg/ml.

In some embodiments, the total flavonoid concentration in a topical formulation provided herein may be 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 30 mg/ml, 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 250 mg/ml, 300 mg/ml, 350 mg/ml, 400 mg/ml, 450 mg/ml, or 500 mg/ml.

In some embodiments, the concentrations of flavonoids in a topical formulation provided herein may be adjusted depending on the phase of wound healing. For example, it has been discovered that a high level of quercetin (e.g., 10 mg/ml to 50 mg/ml) may be desirable during the granulation phase due to its effects on VEGF and TGF-beta. In comparison, the levels of diosmin may remain high (e.g., 10 mg/ml to 50 mg/ml) throughout all phases of the healing cascade.

The cannabinoid(s), terpene(s), and flavonoid(s) used in a topical formulation provided herein may be extracted from natural plants or genetically modified host cells (e.g., yeast cells), or may be synthesized. Terpenes or flavonoids may be extracted from non-cannabis plants such as fruits and vegetables. When the cannabinoid(s), terpene(s), or flavonoid(s) is extracted from a source, the amount of solvent(s) in the extract or the concentration of the cannabinoid, terpene or flavonoid in the extract may vary. For example, an extract may comprise one or more solvents (e.g., an oil or a medium-chain triglyceride), or may be substantially free of any solvent (i.e, containing no detectable level of a solvent). In another example, an extract may be substantially pure (e.g., the concentration of the cannabinoid, terpene or flavonoid in the extract is more than 99% wt).

In some embodiments, a topical formulation provided herein may include a liquid carrier selected for instillation of the topical formulation onto an integumentary wound. The term "liquid carrier" is generally understood to include any carrier that is liquid at ambient temperatures and in which one or more active agents are carried in, dispersed in, or dissolved in. A liquid carrier may be in a form of a viscous liquid, paste, an emulsion or a gel. As can be understood by those skilled in the art, the liquid carrier should be safe for direct application to the integumentary wound, and should avoid or limit irritation or inflammation, or increasing pain level. For example, an alcohol-based carrier would not be suitable for instilling a topical formulation provided herein to the wound bed as it would cause necrosis, pain and irritation at the wound site.

Within the context of the present disclosure, the term "instillation" refers to gradual application or administration of a topical formulation onto a wound bed of a subject. Instillation of a topical formulation provided herein may be carried out by modes of application that include, but are not limited to, dropping, spraying, diffusing, dispersing, squirting, and spreading.

In some embodiments, a suitable liquid carrier may include an aloe vera gel, ointment, or cream; a hyaluronic acid gel, ointment, or cream; a vegetable oil (such as olive oil or sunflower oil); a medium-chain triglyceride; pluronic lecithin organogel; a transdermal base comprising liposomal components; normal saline; or a mixture or combination thereof. In some embodiments, the liquid carrier may be an aloe vera gel. In some embodiments, the liquid carrier may include a mixture or combination of an aloe vera gel and a hyaluronic acid gel. In some embodiments, the liquid carrier may be sunflower oil. In some embodiments, the liquid carrier may be a transdermal base comprising a liposomal component. Examples of transdermal bases comprising liposomal components are LIPODERM™, a family of transdermal bases that are available exclusively from Professional Compounding Centers of America (PCCA). LIPODERM™ is an elegant alternative to traditional pluronic lecithin organogel (PLO) and contains a proprietary liposomal component to increase the permeation of a variety of active pharmaceutical ingredients (APIs).

Medium-chain triglycerides are triglycerides which include a glycerol backbone and a number of fatty acids, where two or three of the fatty acids have an aliphatic tail of 6 to 12 carbon atoms.

Other than cannabinoids, terpenes and flavonoids, which may be considered active agents, a topical formulation provided herein may include one or more additional active agents in some embodiments. The term "active agent" is generally understood to mean an active pharmaceutical ingredient.

Examples of active agents include active herbal extracts, analgesics, local anesthetics, antiepileptics, antiallergic agents, antibacterials, antibiotics, antiburn agents, anticancer agents, antidermatitis agents, antiedemics, antihistamines, antihelminths, antihyperkeratolyte agents, antiinflammatory agents, antiirritants, antimicrobials, antimycotics, antiproliferative agents, antioxidants, antipruritics, antpsoriatic agents, antirosacea agents, antiseborrheic agents, antiseptic, antiswelling agents, antiviral agents, antiyeast agents, astringents, topical cardiovascular agents, chemotherapeutic agents, corticosteroids, dicarboxylic acids, disinfectants, fungicides, hormones, hydroxy acids, immunosuppressants, immunoregulating agents, insecticides, insect repellents, keratolytic agents, lactams, metals, metal oxides, mitocides, neuropeptides, non-steroidal anti-inflammatory agents, oxidizing agents, pediculicides, photodynamic therapy agents, retinoids, sanatives, scabicides, vasoconstrictors, vasodilators, vitamins (e.g., vitamin C) and associated derivatives, minerals, wound healing agents and wart removers.

In some embodiments, topical formulations provided herein may further comprise one or more of an anti-inflammatory agent, a wound healing agent, an anti-oxidizing agent, and an anti-microbial agent.

Topical formulations provided herein may further comprise at least one excipient that does not interfere with the effectiveness or the biological activities of active agents and is not toxic to the subject to which topical formulations provided herein are applied.

Suitable excipients may include preservatives; thickening agents; buffers; isotonic agents; wetting, solubilizing, and emulsifying agents; acidifying agents; alkalinizing agents; carrying agents; chelating agents; complexing agents; solvents; suspending or viscosity-increasing agents; oils; penetration enhancers; polymers; stiffening agents; proteins; carbohydrates; and bulking agents.

Methods of Preparation

An exemplary method of preparing topical formulations provided herein comprises decanting a liquid carrier described herein into a vessel such as a container; adding one or more flavonoids, one or more cannabinoids, and one or more terpenes sequentially to the liquid carrier; and mixing the one or more flavonoids, one or more cannabinoids, and one or more terpenes in the liquid carrier, for example, by shaking the vessel. The method may be carried out in a sterile environment using sterile technique and equipment. The method may also be carried out in a dark environment (e.g., the vessel being covered with dark tape) in order to protect topical formulations from light.

A topical formulation provided herein may also be prepared from a kit provided herein by mixing the materials of the kit following the instructions contained within the kit. For example, one or more flavonoids may be pre-mixed with a liquid carrier and contained in one container in a kit, and one or more cannabinoids and one or more terpenes may be added separately to constitute the topical formulation following the instructions.

Wound Dressings

There are many known topical wound dressings for use in the treatment of wounds or other openings at a physiological target site on a human or animal body which is exuding blood or other bodily fluids. For example, a wound dressing may be selected from wound dressings described in Dhivya S. et al. Biomedicine (Taipei) 2015 Dec; 5(4):24-28.

In an embodiment disclosed herein, a wound dressing or a selected sequence of wound dressings may be used for or after application of a topical formulation to the integumentary wound. A suitable wound dressing may include a wound contact layer. The wound dressing may take the form of a gauze, a bandage, a pad, a foam dressing, a film dressing, a patch, or the like. In some embodiments, the wound contact layer may include a material or layer sold under the trademark JELONET™ or PROFORE WCL™ JELONET™ is a sterile paraffin tulle gras dressing made from open weave gauze and has interlocking threads which minimise fraying when the dressing is cut to shape. PROFORE WCL™ is a 14cm × 20cm (5 1/2" × 8") dressing made of knitted viscose rayon.

In some embodiments, a wound dressing described above may be used separately from a formulation described herein. For example, the formulation and the wound dressing may be applied to an integumentary wound sequentially. In some embodiments, a wound dressing described above may be used concurrently with a formulation described herein. For example, the formulation may be integrated with the contact layer in the wound dressing before use such that the formulation is releasable from the contact layer at a suitable rate after the wound dressing is applied onto an integumentary wound.

Methods and Uses

Topical formulations and wound dressings provided herein may be useful for the treatment of an integumentary wound of a subject. Without being limited by any particular theory, it is expected that a topical formulation as described herein may promote wound healing by synergistically stimulating granulation tissue growth and promoting epithelialization, through one or more epigenetic mechanisms including interaction with the endocannabinoid system.

The endocannabinoid system (ECS) is ubiquitous throughout the human body and has recently been found to have a significant representation throughout the integumentary system, both cutaneous membranes and mucous membranes. The ECS is principally composed of cannabinoid receptors (CB1 and CB2), endogenous ligands (AEA and 2-AG), biosynthetic pathways (NAPE and DAGL), and degradation pathways (FAAH and MAGL). Both cannabinoids and non-cannabinoids, such as terpenes and flavonoids, are capable of complex direct and indirect interactions with the ECS of the integumentary system.

The terms "treat," "treating," or "treatment of' are used herein in their broad senses unless otherwise specifically indicated in the particular context, and results of a treatment may generally include reversing, alleviating, or inhibiting the progress of an indicated disorder or condition, or one or more symptoms of the disorder or condition.

As used herein, the term "individual" or "subject" means an animal; for example, a mammal such as a human. Mammals also include farm animals, sport animals, companion animals, primates, horses, dogs, cats, mice and rats.

In some embodiments, a method of treating an integumentary wound may include instilling a topical formulation provided herein, for example, a solution or colloid, onto an integumentary wound of a subject. Instillation may be carried out by dropping, spraying, diffusing, dispersing, squirting, or spreading the topical formulation. An applicator may be used for instillation. Examples of applicators include a dropper, a nebulizer, an impregnated gauze sheet, a syringe, and a cotton swap. Post instillation, the topical formulation may cover one or more areas within the integumentary wound, or may cover the entire integumentary wound (including the wound edge), or may cover the entire integumentary wound as well as an area adjacent to an edge the integumentary wound (e.g., the periwound area).

In some embodiments, a method of treating an integumentary wound may include instilling a topical formulation provided herein, for example, a solution or colloid, onto both an integumentary wound of a subject and a periwound area around the integumentary wound. The periwound area is typically limited to the integument surrounding an open wound within about 4 cm of the wound edge, but may extend beyond the 4 cm limit depending on the extent of the damages present. For example, as can be appreciated by those skilled in the art, the periwound area may be proportionate to the size of the open wound, and may cover any skin area that is at risk of further breakdown. It is recognized that tissues within the periwound area may exhibit pathophysiologic features such as inflammation, edema, vasoconstriction, lymphatic obstruction, reduced oxygen tensions, and acidosis from reduced cellular chemotaxis ("leukocyte entrapment"). Topical formulations suitable for applications to both an integumentary wound of a subject and a periwound area around the integumentary wound may comprise both THC and CBD, which are compounded in either PLO or liposomes. Treatment according to the method of instilling a topical formulation provided herein onto both an integumentary wound of a subject and a periwound area around the integumentary wound may promote vasodilation and/or oxygenation.

Furthermore, treatment of the periwound area is expected to promote wound closure and healing, as well as prevent tissues within the periwound area from deteriorating and enlarging.

In some embodiments, a wound dressing or a selected sequence of wound dressings may be applied onto the integumentary wound after instilling a topical formulation provided herein. The wound dressing may comprise a wound contact layer that includes a material or layer sold under the trademark JELONET™ or PROFORE WCL™ The wound dressing may be a foam dressing or a film dressing.

In some embodiments, a topical formulation disclosed herein may be first applied to a wound dressing and the wound dressing is then applied onto an integumentary wound of a subject.

In some embodiments, topical formulations provided herein are used for the treatment of an integumentary wound of a subject. Topical formulations provided herein may be instilled onto the integumentary wound. Instillation may be carried out by dropping, spraying, diffusing, dispersing, squirting, or spreading the formulation.

In some embodiments, a wound dressing or a selected sequence of wound dressings is used for application onto the integumentary wound after a topical formulation provided herein has been first applied to the integumentary wound. The wound dressing may comprise a wound contact layer that includes a material or layer sold under the trademark JELONET™ or PROFORE WCL™. The wound dressing may be a foam dressing or a film dressing.

In some embodiments, topical formulations provided herein are used in combination with existing therapies for wound healing. For example, topical formulations provided herein may be instilled onto an integumentary wound of a subject during Negative Pressure Wound Therapy (NPWT). In some embodiments, a topical formulation provided herein comprising normal saline as the liquid carrier is delivered to an integumentary wound through NPWT foam dressing. The formulation may be removed together with exudate from the integumentary wound. Using a NPWT canister, fresh formulation may be instilled onto the integumentary wound and subsequently removed together with exudate in a cyclic manner.

It has been surprisingly discovered by the present inventor that certain combination therapies can exhibit synergistic effects. For example, it has been found out that combining topical formulations provided herein with Electrical Stimulation Therapy (EST) resulted in better healing efficacy. Without being limited by any particular theory, it is expected that while cannabinoids and non-cannabinoids comprised in topical formulations provided herein bind to extracellular receptors on the membrane of intact cells at an integumentary wound, EST promotes the process of "electroporation" (i.e., creating openings within cell membranes), thereby allowing cannabinoids and non-cannabinoids to enter cells and interact with the intracellular binding sites of cannabinoid receptors (both extracellular and intracellular binding are expected to continue after electroporation is reversed). In some embodiments, there is provided a combination therapy comprising applying a topical formulation provided herein to an integumentary wound, and then applying an electric pulse generated by an electrical stimulation generator to the integumentary wound (for example, via electrodes attached to skin surrounding the wound).

The integumentary wound to be treated may be acute. Alternatively, the integumentary wound to be treated may be chronic, stalled, recalcitrant, or a combination thereof. For example, the integumentary wound may be caused by a skin ulcer, a burn (a radiation therapy burn, a chemical burn, a thermal burn, or a sun burn), or a traumatic abrasion or skin tear. Possible skin ulcers include diabetic ulcers (e.g., neuroischemic diabetic foot ulcer or diabetic dermopathies such as Necrobiosis Lipoidica Diabeticorum), pressure injury ulcer, arterial leg ulcer, venous leg ulcer, or arterial ulcer (e.g., arterial ulcer with critical ischemia).

The integumentary wound may be iatrogenic (drug induced) or caused by a skin disease or systemic condition. For example, the skin disease or condition may be Skin Cancer (Primary Neoplasms & Metastatic Neoplasms), Integumentary Ulcers and Erosions caused by microbes (e.g., a bacterium, fungus, virus, and mycobacterium), Pyoderma Gangrenosum, Leukocytoclastic Vasculitis, Cryoglobulinemia, Cryofibrinogenemia, Antiphospholipid Syndrome, Sickle Cell Disease, Lichen Simplex Chronicus, Bowen's Disease, Martorell's Ulcer, Calciphylaxis, Allergic Dermatitis, Psoriasis, Epidermolysis Bullosa, Pemphigus, Bullous Pemphigoid, Behcet's Disease, Rheumatoid arthritis, Systemic Lupus Erythematosis, Scleroderma, Wegeners Granulomatosis, or Hidradenitis Suppurativa.

The subject under treatment may be a human, or an animal.

In some embodiments, treatment of an integumentary wound may provide concurrent or separate analgesia, opioid-sparing effect, antimicrobial activity, and scar tissue mitigation.

In some embodiments, treatment of an integumentary wound may stimulate granulation tissue growth. For example, tests have shown that at least 33% of an integumentary wound may be granulated within 7 days after instillation of a topical formulation provided herein, or at least 66% of an integumentary wound may be granulated within 14 days after instillation of a topical formulation as disclosed herein.

In some embodiments, treatment of an integumentary wound may prevent scar (e.g., keloid) formation and/or completely close the wound.

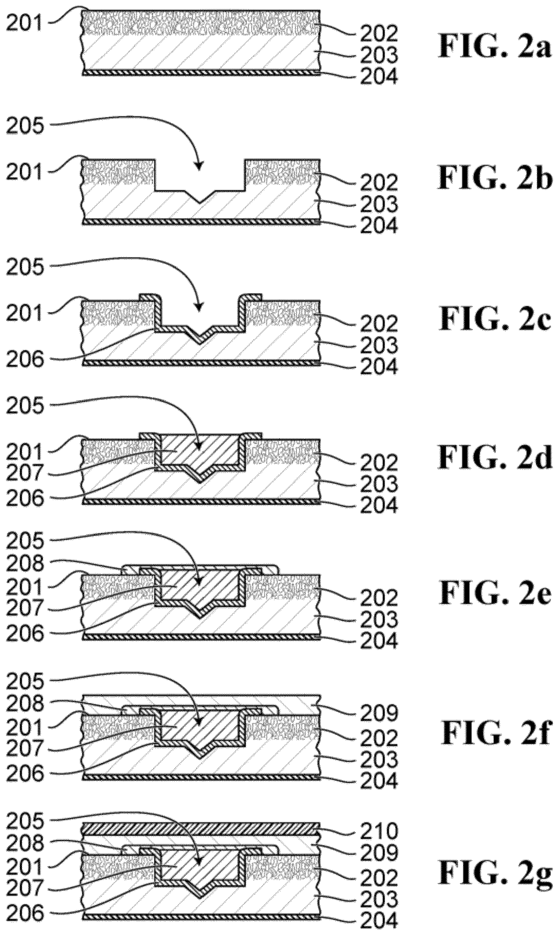

In a particular embodiment, an integumentary wound may be treated as follows, with reference to FIG.2.

FIG.2a illustrates an intact skin, comprising epidermis 201, dermis 202, subcutaneous tissue 203 and superficial fascia 204.

As illustrated in FIG. 2b, at stage 1 of the treatment, the integumentary wound 205 is assessed for treatment. An initial formulation is prepared or obtained based on the assessment. The initial formulation may be a formulation as described herein with one or more adjustments based on the assessment. For example, if the wound to be treated is in the inflammatory phase, the concentrations of cannabinoids, using a combination of THC and CBD as an example, may be adjusted as follows: 2 mg/ml to 10 mg/ml of THC, and 5 mg/ml to 20 mg/ml of CBD.

At stage 1, the wound bed and optionally its periwound area of the integumentary wound 205 are also prepared for treatment. The wound bed may be prepared in any suitable manner or using any suitable technique. Example wound preparation techniques are provided in Sibbald RG. et al. Journal of Cutaneous Medicine and Surgery, 2013, volume 17, issue 4, suppl. pages S12-S22.

At stage 2, the initial formulation 206 is instilled directly onto the wound bed and optionally its periwound area of the integumentary wound 205. As depicted in FIG. 2c, a layer of the formulation 206 is applied on the top surface of subcutaneous tissue of the open wound, which forms the wound bed, as well as the periwound area. The application of the formulation may be carried out depending on the form of the formulation. For example, if the initial formulation is oil-based, the formulation may be dropped or sprayed over the wound bed. If the initial formulation is a gel, the formulation may be spread onto the wound bed such as with sterile cotton tipped applicator.

At stage 3 depicted in FIG. 2d, a wound dressing including a wound contact layer 207 is applied on top of the formulation layer 206. As can be appreciated, in some cases, a selected sequence of wound dressings may be applied over the wound bed covering the applied formulation. The wound contact layer may be JELONET or PROFORE WCL. The contact layer 207 may optionally include a selected formulation as described herein, which is integrated with the contact layer in a manner such that the formulation is releasable onto the wound bed of the integumentary wound 205 when the contact layer 207 is in contact with the wound bed.

At optional stage 4 depicted in FIG. 2e, a wound cavity filler 208, such as calcium alginate or hydrofibers, may be used to fill the space above the wound dressing if needed.

At optional stage 5 depicted in FIG. 2f, an absorptive layer 209, which may include MESORB™ or foam, or the like, may be applied on top of the wound dressing and optionally the cavity filler.

At optional stage 6 depicted in FIG. 2g, a compression therapy 210 may be optionally conducted. The compression therapy may be elastic or inelastic compression therapy.

The formulation may be applied one to four times daily over a period of several days to a number of weeks.

The wound is re-assessed from time to time over the period of treatment, and depending on the development of the wound healing process, the formulation to be subsequently applied to the wound may be adjusted.

For example, when the treated wound has progressed to the proliferative phase and continues into the remodeling phase, a subsequent formulation may be prepared or obtained, which may have a reduced concentration of cannabinoids, and an increased concentration of terpenes, especially terpenes that are agonists for CB2 receptors. In some embodiments, a topical formulation provided herein comprises beta-caryophyllene, and the concentration of beta-caryophyllene may be 30 mg/ml to 60 mg/ml when the formulation is used during the inflammatory phase, and may be subsequently increased to 50 mg/ml to 500 mg/ml when the inflammatory phase of wound healing is completed. The subsequent formulation may be applied directly to the wound bed one to four times daily over a period, such as few days to a number of weeks. This formulation may be used through the proliferative and remodeling phases until the wound is completely healed.

In a different embodiment, treatment with a topical formulation described herein may be applied in one or two phases of the wound healing process. For example, a treatment may start in the proliferative phase, instead of the inflammatory phase.

In general, the adjustment to the contents of the topical formulation, or the selected wound dressing, may be selected depending on the nature of the integumentary wound, or as the integumentary wound progresses through different phases of wound healing. For example, a higher concentration of a psychoactive cannabinoid such as THC in the initial formulation may be beneficial for pain management in the inflammatory phase and/or may be desirable for wounds having a low oxygen level. However, after the inflammatory phase, the concentration of the psychoactive cannabinoid may be reduced to avoid inhibition of keratinocyte differentiation. In another example, a terpene that is an agonist for CB2 receptors may be beneficially included in the formulation(s) applied to the wound after the inflammatory phase has completed, or the concentration of such a terpene in the formulation(s) may be increased during the proliferative and remodeling phases, as such a terpene may promote re-epithelialization and remodeling.

The formulations may also be adjusted depending on the natures of the wound and one or more conditions of the treated subject.

In a specific example, a base topical formulation may include:

(a) 2.3 mg/ml of cannabidiol (CBD) and 1.0 mg/ml of tetrahydrocannabinol (THC);

(b) 81.5 mg/ml of beta-caryophyllene and 28.4 mg/ml of linalool; and

(c) 16.7 mg/ml of micronized diosmin and 16.7 mg/ml of micronized quercetin.

The formulations to be used at different phases of treatment and would healing for different subjects under treatment may be adjusted from this base formulation as follows.

If the subject under treatment has significant levels of venous lymphedema in the lower limbs, the concentration of diosmin is increased to 10 mg/ml to 50 mg/ml.

During the inflammatory phase of wound healing, the concentration of THC is increased to 2 mg/ml to 10 mg/ml, and the concentration of CBD is increased to 5 mg/ml to 20 mg/ml, since wound pain is expected to be most intense during this phase and increased CBD and THC may help reducing pain.

During the re-epithelial and remodeling phases, the concentration of THC is maintained within the range of 0 mg/ml to 5 mg/ml, the concentration of CBD is maintained within the range of 0.1 mg/ml to 20 mg/ml, and the concentration of beta-caryophyllene is maintained within the range of 50 mg/ml to 500 mg/ml, which is expected to avoid inhibition of keratinocyte differentiation and to promote re-epithelialization and remodeling. During the granulation phase, the concentration of quercetin is increased to 10 mg/ml to 50 mg/ml, which is expected to have effects on both VEGF and TGF-beta.

The base formulation may be further adjusted in some embodiments. For example, linalool may be substituted with another monoterpene such as alpha-bisabolol, thymol, alpha-terpineol, and genipin, or a triterpene such as astragaloside and asiaticoside. THC may be substituted entirely by THCV. The THC, CBD, and THCV may be decarboxylated, or may be substituted by their respective noncarboxylated natural acid formats.

Kits

In some embodiments, a kit may be provided, which include a container containing a topical formulation as disclosed herein, or a number of containers containing materials for preparing the topical formulation. The kit may also include instructions for treating an integumentary wound using the topical formation including dosage and how the formulation may be applied or instilled onto the wound bed. When separate containers are provided in the kit, and depending on the contents in these containers, the kit may also include instructions for preparing a topical formulation, or formulations with different concentrations of active ingredients, from the materials included in the kit and optionally other materials such as a liquid carrier or other additives. The kit may also include a liquid carrier as described elsewhere herein. The kit may further include an applicator for applying the topical formulation to the wound bed, and may include specific instructions on how to use the applicator.

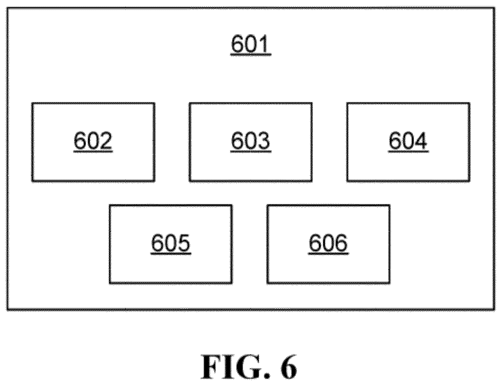

In an embodiment, a topical formulation may be prepared or obtained from a kit comprising (a) one or more cannabinoids; (b) one or more terpenes; (c) one or more flavonoids; (d) a liquid carrier selected for instillation of the topical formulation onto an integumentary wound; and (e) instructions, wherein at least one of (a), (b) and (c) is not mixed with (d) in the kit, and wherein the instructions comprise information allowing all of (a), (b) and (c) be mixed with (d) at selected concentrations disclosed herein. The kit may include separate containers (see, e.g., the kit 601 depicted in FIG. 6 comprising a first container 602 comprising (a), a second container 603 comprising (b), a third container 604 comprising (c), a fourth container 605 comprising (d), and a fifth container 606 comprising (e)) or instructions for providing or preparing more than one formulation with different concentrations for one or more of (a), (b) and (c).

In some embodiments, a kit may include a container containing an instillate or a formulation provided herein. The formulation may be in form of oil, gel, paste or the like as described above. The container may be, for example, a liquid bottle or a paste tube depending on the physical form of the formulation. In other embodiments, a kit may include a plurality of containers containing materials for forming an instillate or a formulation provided herein. The kit may further comprise at least one of instructions for applying the instillate or formulation directly to a wound bed and optionally a periwound area around the integumentary wound; instructions for using the instillate or formulation to treat an integumentary wound according to the methods or uses provided herein; and instructions for using the materials in the plurality of containers to prepare the instillate or formulation according to the methods of preparation provided herein. Optional components of a kit may include one or more applicators (such as droppers, sprayers, gauze sheets, and cotton tipped applicators) for applying the instillate or formulation onto an open wound bed and a periwound area around the integumentary wound, and one or more wound dressings as described herein. The one or more applicators may be sterilized and contained in a sealed sterile packaging.

Embodiments

Particular embodiments of the invention include, without limitation, the following:

1. A topical formulation comprising:

(a) 0.1 mg/ml to 40 mg/ml of one or more cannabinoids;

(b) 25 mg/ml to 1000 mg/ml of one or more terpenes; and

(c) 10 mg/ml to 500 mg/ml of one or more flavonoids.

2. The topical formulation of paragraph 1, wherein the one or more cannabinoids comprise cannabidiol.

3. The topical formulation of paragraph 2, wherein the one or more cannabinoids further comprise at least one of cannabinol, cannabigerol, cannabichromene, and tetrahydrocannabivarin.

4. The topical formulation of paragraph 2 or 3, wherein the one or more cannabinoids further comprise tetrahydrocannabinol.

5. The topical formulation of paragraph 2 or 3, wherein the one or more cannabinoids do not comprise tetrahydrocannabinol.

6. The topical formulation of paragraph 1, wherein the one or more cannabinoids do not comprise tetrahydrocannabinol.

7. The topical formulation of any one of paragraphs 1 to 6, wherein the one or more terpenes comprise beta-caryophyllene.

8. The topical formulation of any one of paragraphs 1 to 7, wherein the one or more terpenes comprise beta-caryophyllene and a monoterpene such as linalool, thymol, alpha-bisabolol, alpha-terpineol and genipin or a triterpene such as astragaloside and asiaticoside.

9. The topical formulation of any one of paragraphs 1 to 8, wherein the one or more flavonoids comprise quercetin.

10. The topical formulation of any one of paragraphs 1 to 8, wherein the one or more flavonoids comprise at least one of diosmin, quercetin, and hesperidin, or comprise at least one of kaempferol, apigenin, and quercetin, or comprise kaempferol, apigenin, diosmin, hesperidin, and quercetin.

11. The topical formulation of any one of paragraphs 1 to 10, wherein the one or more cannabinoids, terpenes, or flavonoids are extracted from plants or genetically modified host cells, or are synthetic.

12. The topical formulation of any one of paragraphs 1 to 11, which comprises 0.1 mg/ml to 20 mg/ml of cannabidiol and 0 mg/ml to 5 mg/ml of tetrahydrocannabinol.

13. The topical formulation of any one of paragraphs 1 to 11, which comprises 5 mg/ml to 20 mg/ml of cannabidiol and 2 mg/ml to 10 mg/ml of tetrahydrocannabinol.

14. The topical formulation of paragraph 12 or 13, wherein tetrahydrocannabinol is replaced with tetrahydrocannabivarin.

15. The topical formulation of any one of paragraphs 1 to 14, which comprises 50 mg/ml to 500 mg/ml of the one or more terpenes.

16. The topical formulation of any one of paragraphs 1 to 15, which comprises 20 mg/ml to 200 mg/ml of the one or more flavonoids.

17. The topical formulation of any one of paragraphs 1 to 14, which comprises 30 mg/ml to 60 mg/ml of beta-caryophyllene and 10 mg/ml to 30 mg/ml of linalool.

18. The topical formulation of any one of paragraphs 1 to 14, which comprises 50 mg/ml to 500 mg/ml of beta-caryophyllene and 10 mg/ml to 150 mg/ml of linalool.

19. The topical formulation of paragraph 17 or 18, wherein linalool is replaced with a monoterpene such as linalool, thymol, alpha-bisabolol, alpha-terpineol and genipin or a triterpene such as astragaloside and asiaticoside.

20. The topical formulation of any one of paragraphs 1 to 19, which comprises 10 mg/ml to 50 mg/ml of diosmin and 10 mg/ml to 50 mg/ml of quercetin.

21. The topical formulation of any one of paragraphs 1 to 20, wherein the one or more flavonoids are micronized.

22. The topical formulation of any one of paragraphs 1 to 21, wherein the one or more flavonoids are in the form of dry powders.

23. The topical formulation of paragraph 1, comprising (a) 10 mg/ml to 20 mg/ml of cannabidiol and at least one of cannabinol, cannabigerol, cannabichromene, and tetrahydrocannabivarin; (b) 0.1 mg/ml to 0.2 mg/ml of at least one of linalool, thymol, alpha-bisabolol, and myrcene; and (c) 100 mg/ml to 200 mg/ml of at least one of kaempferol, apigenin, diosmin, hesperdin, and quercetin.

24. The topical formulation of paragraph 1, comprising (a) 5 mg/ml to 30 mg/ml of cannabidiol and 2 mg/ml to 10 mg/ml of tetrahydrocannabinol or tetrahydrocannabivarin; (b) 30 mg/ml to 60 mg/ml of beta-caryophyllene and 10 mg/ml to 30 mg/ml of linalool; and (c) 10 mg/ml to 30 mg/ml of diosmin and 10 mg/ml to 30 mg/ml of quercetin.

25. The topical formulation of paragraph 1, comprising (a) 0.1 mg/ml to 20 mg/ml of cannabidiol and 0 mg/ml to 5 mg/ml of tetrahydrocannabinol or tetrahydrocannabivarin; (b) 50 mg/ml to 500 mg/ml of beta-caryophyllene and 10 mg/ml to 150 mg/ml of linalool; and (c) 0 mg/ml to 50 mg/ml of diosmin and 10 mg/ml to 50 mg/ml of quercetin.

26. The topical formulation of paragraph 1, comprising (a) 2.3 mg/ml of cannabidiol and 1.0 mg/ml of tetrahydrocannabinol; (b) 81.5 mg/ml of beta-caryophyllene and 28.4 mg/ml of linalool; and (c) 16.7 mg/ml of micronized diosmin and 16.7 mg/ml of micronized quercetin.

27. The topical formulation of paragraph 1, comprising (a) 2.6 mg/ml of cannabidiol; (b) 118 mg/ml of beta-caryophyllene; (c) 19.6 mg/ml of micronized diosmin, 21.7 mg/ml of micronized quercetin, and 2.2 mg/ml of hersperidin; and (d) aloe vera gel and hyaluronic acid gel

28. The topical formulation of any one of paragraphs 1 to 27, which is in the form of solution, lotion, cream, ointment, gel, emulsion, liposome, foam, powder, impregnated gauze sheet, tulle, vapor or paste for application to a cutaneous wound; or in the form of aerosolized spray for nasal or oral application to a cutaneous wound; or in the form of suppository for rectal or vaginal application to a cutaneous wound.

29. The topical formulation of any one of paragraphs 1 to 27, which is a solution or colloid.

30. The topical formulation of paragraph 29, which further comprises a liquid carrier selected for instillation of the solution or colloid onto an integumentary wound.

31. The topical formulation of paragraph 30, wherein the liquid carrier comprises an aloe vera gel, a hyaluronic acid gel, a vegetable oil, a medium-chain triglyceride, pluronic lecithin organogel, a transdermal base comprising a liposomal component, or normal saline.

32. The topical formulation of paragraph 31, wherein the vegetable oil is olive oil or sunflower oil.

33. The topical formulation of any one of paragraphs 30 to 32, wherein the liquid carrier is sunflower oil.

34. The topical formulation of paragraph 30 or 31, wherein the liquid carrier is pluronic lecithin organogel or a transdermal base comprising a liposomal component.

35. The topical formulation of paragraph 34, which is in the form of cream.

36. The topical formulation of paragraph 30 or 31, wherein the liquid carrier comprises the aloe vera gel.

37. The topical formulation of paragraph 30 or 31, wherein the liquid carrier comprises the aloe vera gel and the hyaluronic acid gel.

38. The topical formulation of any one of paragraphs 1 to 37, which further comprises one or more additional active agents.

39. The topical formulation of paragraph 38, wherein the one or more active agents comprise at least one of an anti-inflammatory agent, a wound healing agent, an anti-oxidizing agent, and an anti-microbial agent.

40. The topical formulation of any one of paragraphs 1 to 39, which further comprises an excipient.

41. A wound dressing comprising a wound contact layer and the topical formulation of any one of paragraphs 1 to 40 integrated with the contact layer.

42. The wound dressing of paragraph 41, wherein the wound contact layer comprises a paraffin gauze dressing or a dressing made of knitted viscose rayon.

43. The wound dressing of paragraph 41 or 42, which is a foam dressing.

44. The wound dressing of paragraph 41 or 42, which is a film dressing.

45. A method comprising instilling the topical formulation of any one of paragraphs 1 to 40 onto an integumentary wound of a subject.

46. The method of paragraph 45, wherein the step of instilling covers a portion of the integumentary wound.

47. The method of paragraph 45, wherein the step of instilling covers the entire integumentary wound.

48. The method of paragraph 45, wherein the step of instilling covers the entire integumentary wound and an area adjacent to an edge the integumentary wound.

49. The method of paragraph 48, wherein the area adjacent to the edge of the integumentary wound is a periwound area around the integumentary wound.

50. A method comprising instilling the topical formulation of any one of paragraphs 1 to 40 onto an integumentary wound of a subject and a peri wound area around the integumentary wound.

51. The method of any one of paragraphs 45 to 50, wherein the instilling comprises dropping, spraying, diffusing, dispersing, squirting, or spreading the formulation.

52. The method of any one of paragraphs 45to 51, further comprising applying a wound dressing comprising a wound contact layer onto the integumentary wound after instillation of the topical formulation.

53. The method of paragraph 52, wherein the wound contact layer comprises a paraffin gauze dressing or a dressing made of knitted viscose rayon.

54. The method of paragraph 52 or 53, wherein the wound dressing is a foam dressing.

55. The method of paragraph 52 or 53 wherein the wound dressing is a film dressing.

56. A method comprising applying the wound dressing of any one of paragraphs 41 to 44 onto an integumentary wound, and optionally a periwound area around the integumentary wound, of a subject.

57. The method of any one of paragraphs 45 to 56, wherein the integumentary wound is acute, or is chronic, stalled, recalcitrant, or a combination thereof.

58. The method of any one of paragraphs 45 to 57, wherein the integumentary wound is caused by a skin ulcer, a burn, or a traumatic abrasion or skin tear.

59. The method of paragraph 58, wherein the skin ulcer is a diabetic ulcer, a pressure ulcer, an arterial leg ulcer, a venous leg ulcer, or an arterial ulcer.

60. The method of any one of paragraphs 45 to 59, wherein the integumentary wound is caused by a skin disease or condition.

61. The method of paragraph 60, wherein the skin disease or condition is Skin Cancer (Primary Neoplasms & Metastatic Neoplasms), Integumentary Ulcers and Erosions caused by microbes (e.g., a bacterium, fungus, virus, and mycobacterium), Pyoderma Gangrenosum, Leukocytoclastic Vasculitis, Cryoglobulinemia, Cryofibrinogenemia, Antiphospholipid Syndrome, Sickle Cell Disease, Lichen Simplex Chronicus, Bowen's Disease, Martorell's Ulcer, Calciphylaxis, Allergic Dermatitis, Psoriasis, Epidermolysis Bullosa, Pemphigus, Bullous Pemphigoid, Behcet's Disease, Rheumatoid arthritis, Systemic Lupus Erythematosis, Scleroderma, Wegeners Granulomatosis, or Hidradenitis Suppurativa.

62. The method of any one of paragraphs 45 to 61, wherein the subject is a human.

63. The method of any one of paragraphs 45 to 61, wherein the subject is an animal.

64. The method of any one of paragraphs 45 to 63, which has an analgesic, anti-inflammatory, anti-microbial, or anti-pruritic effect.

65. The method of any one of paragraphs 45 to 64, which has an opioid-sparing effect.

66. The method of any one of paragraphs 45 to 65, which stimulates granulation tissue growth.

67. The method of paragraph 66, wherein at least 33% of the integumentary wound is granulated within 7 days.

68. The method of paragraph 66, wherein at least 66% of the integumentary wound is granulated within 14 days.

69. The method of any one of paragraphs 45 to 68, wherein the topical formulation is effective for reducing scar formation.

70. The method of any one of paragraphs 45 to 69, further comprising adjusting the topical formulation depending on the nature of the integumentary wound, or as the integumentary wound progresses through different phases of wound healing.

71. The method of paragraph 70, wherein adjusting the topical formulation comprises reducing the concentrations of the one or more cannabinoids in the topical formulation, when the inflammatory phase of wound healing is completed.

72. The method of paragraph 71, wherein when the topical formulation comprises cannabidiol and tetrahydrocannabinol, the concentration of cannabidiol is 5 mg/ml to 20 mg/ml before adjustment and is reduced to 0.1 mg/ml to 20 mg/ml when the inflammatory phase of wound healing is completed, and the concentration of tetrahydrocannabinol is 2 mg/ml to 10 mg/ml before adjustment and is reduced to 0 mg/ml to 5 mg/ml when the inflammatory phase of wound healing is completed.

73. The method of paragraph 70, wherein adjusting the topical formulation comprises increasing the concentrations of the one or more terpenes in the topical formulation, when the inflammatory phase of wound healing is completed.

74. The method of paragraph 73, wherein when the topical formulation comprises beta-caryophyllene, the concentration of beta-caryophyllene is 30 mg/ml to 60 mg/ml before adjustment and is increased to 50 mg/ml to 500 mg/ml when the inflammatory phase of wound healing is completed.

75. The method of paragraph 70, wherein adjusting the formulation comprises increasing the concentrations of the one or more flavonoids in the topical formulation, when the wound healing is in the granulation phase.

76. The method of paragraph 75, wherein the topical formulation comprises 10 mg/ml to 50 mg/ml quercetin when the wound healing is in the granulation phase.

77. The method of any one of paragraphs 45 to 76, further comprising treating the integumentary wound with an additional therapy for wound healing.

78. The method of paragraph 77, wherein the additional therapy is negative pressure wound therapy.

79. The method of paragraph 77, wherein the additional therapy is electrical stimulation therapy.

80. Use of the topical formulation of any one of paragraphs 1 to 40 for the treatment of an integumentary wound of a subject.

81. The use of paragraph 80, wherein the solution is for instillation onto the integumentary wound and optionally a periwound area around the integumentary wound.

82. The use of paragraph 81, wherein the instillation comprises dropping, spraying, diffusing, dispersing, squirting, or spreading the topical formulation onto the integumentary wound.

83. The use of any one of paragraphs 80 to 82, further comprising use of a wound dressing comprising a wound contact layer, wherein the wound dressing is for application onto the integumentary wound after the topical formulation.

84. The use of paragraph 83, wherein the wound contact layer comprises a paraffin gauze dressing or a dressing made of knitted viscose rayon.

85. The use of paragraph 83 or 84, wherein the wound dressing material is a foam dressing.

86. The use of paragraph 83 or 84, wherein the wound dressing material is a film dressing.

87. Use of the wound dressing of any one of paragraphs 41 to 44 for the treatment of an integumentary wound of a subject.

88. The use of any one of paragraphs 80 to 87, wherein the integumentary wound is acute, or is chronic, stalled, recalcitrant or a combination thereof.

89. The use of paragraph 88, wherein the integumentary wound is caused by a skin ulcer, a burn, or a traumatic abrasion or skin tear.

90. The use of paragraph 89, wherein the skin ulcer is a diabetic ulcer, a pressure injury ulcer, an arterial leg ulcer, a venous leg ulcer, or an arterial ulcer.

91. The use of any one of paragraphs 80 to 90, wherein the integumentary wound is caused by a skin disease or condition.

92. The use of paragraph 91, wherein the skin disease or condition is Skin Cancer (Primary Neoplasms & Metastatic Neoplasms), Integumentary Ulcers and Erosions caused by microbes (e.g., a bacterium, fungus, virus, and mycobacterium), Pyoderma Gangrenosum, Leukocytoclastic Vasculitis, Cryoglobulinemia, Cryofibrinogenemia, Antiphospholipid Syndrome, Sickle Cell Disease, Lichen Simplex Chronicus, Bowen's Disease, Martorell's Ulcer, Calciphylaxis, Allergic Dermatitis, Psoriasis, Epidermolysis Bullosa, Pemphigus, Bullous Pemphigoid, Behcet's Disease, Rheumatoid arthritis, Systemic Lupus Erythematosis, Scleroderma, Wegeners Granulomatosis, or Hidradenitis Suppurativa.

93. The use of any one of paragraphs 80 to 92, wherein the subject is a human.

94. The use of any one of paragraphs 80 to 92, wherein the subject is an animal.

95. The use of any one of paragraphs 80 to 94, which has an analgesic, anti-inflammatory, anti-microbial, or anti-pruritic effect.

96. The use of any one of paragraphs 80 to 95, which has an opioid-sparing effect.

97. The use of any one of paragraphs 80 to 96, which stimulates granulation tissue growth.

98. The use of paragraph 97 wherein at least 33% of the integumentary wound is granulated within 7 days.

99. The use of paragraph 97, wherein at least 66% of the integumentary wound is granulated within 14 days.

100. The use of any one of paragraphs 80 to 99, which prevents scar formation.

101. The use of any one of paragraphs 80 to 100, which promotes vasodilation and/or oxygenation.

102. The use of any one of paragraphs 80 to 101, comprising adjusting the topical formulation depending on the nature of the integumentary wound, or as the integumentary wound progresses through different phases of wound healing.

103. The use of paragraph 102, wherein adjusting the topical formulation comprises reducing the concentrations of the one or more cannabinoids in the topical formulation, when the inflammatory phase of wound healing is completed.

104. The use of paragraph 103, wherein when the topical formulation comprises cannabidiol and tetrahydrocannabinol, the concentration of cannabidiol is 5 mg/ml to 20 mg/ml before adjustment and is reduced to 0.1 mg/ml to 20 mg/ml when the inflammatory phase of wound healing is completed, and the concentration of tetrahydrocannabinol is 2 mg/ml to 10 mg/ml before adjustment and is reduced to 0 mg/ml to 5 mg/ml when the inflammatory phase of wound healing is completed.

105. The use of paragraph 102, wherein adjusting the topical formulation comprises increasing the concentrations of the one or more terpenes in the topical formulation, when the inflammatory phase of wound healing is completed.

106. The use of paragraph 105, wherein when the topical formulation comprises beta-caryophyllene, the concentration of beta-caryophyllene is 30 mg/ml to 60 mg/ml before adjustment and is increased to 50 mg/ml to 500 mg/ml when the inflammatory phase of wound healing is completed.

107. The use of paragraph 102, wherein adjusting the formulation comprises increasing the concentrations of the one or more flavonoids in the topical formulation, when the wound healing is in the granulation phase.

108. The use of paragraph 107, wherein the topical formulation comprises 10 mg/ml to 50 mg/ml quercetin when the wound healing is in the granulation phase.

109. The use of any one of paragraphs 90 to 108, further comprising an additional therapy for wound healing.

110. The use of paragraph 109, wherein the additional therapy is negative pressure wound therapy.

111. The use of paragraph 109, wherein the additional therapy is electrical stimulation therapy.

112. A kit comprising: a container containing the topical formulation of any one of paragraphs 1 to 40, or a plurality of containers containing materials for forming the topical formulation of any one of paragraphs 1 to 40.

113. The kit of paragraph 112, further comprising instructions for applying the topical formulation directly to an integumentary wound and optionally a periwound area around the integumentary wound.

114. The kit of paragraph 112 or 113, further comprising instructions for using the topical formulation to treat an integumentary wound according to the method of any one of paragraphs 45 to 75.

115. The kit of any one of paragraphs 112 to 114, further comprising instructions for using the materials in the plurality of containers to prepare the topial formulation.

116. The kit of any one of paragraphs 112 to 115, further comprising one or more applicators for applying the topical formulation onto a wound bed.

117. The kit of any one of paragraphs 112 to 116, further comprising one or more wound dressings of any one of paragraphs 41 to 44.

118. A method comprising instilling a topical formulation comprising cannabidiol and a liquid carrier onto an integumentary wound, and optionally the periwound area of the integumentary wound, of a subject.

119. The method of paragraph 118, wherein the topical formulation consists of cannabidiol and the liquid carrier.

120. The method of paragraph 118 or 119, wherein the topical formulation is instilled onto an integumentary wound.

121. The method of any one of paragraphs 118 to 120, wherein the liquid carrier comprises an aloe vera gel, a hyaluronic acid gel, a vegetable oil, a medium-chain triglyceride, pluronic lecithin organogel, or a transdermal base comprising a liposomal component.

122. The method of paragraph 121, wherein the liquid carrier comprises an aloe vera gel or a hyaluronic acid gel.

**EXAMPLES**

Example 1: Preparation of A Topical Formulation of Cannabinoids, Terpenes and Flavonoids

The formulation was prepared in 30ml aliquots using sterile technique. 10 ml of aloe vera gel (purchased under the commercial name "Aloe Gel" from Realaloe Canada, British Columbia, Canada) and 10 ml of hyaluronic acid gel (containing 8 mg/ml of hyaluronic acid; purchased under the commercial name "PRO HA + C" from Naka Professional, Toronto, Canada M9W 5S2) were decanted into a sterile container. The dried powder forms of diosmin (purchased under the commercial name "Diovasc" from Xymogen Inc, 32819 USA), and quercetin (purchased under the commercial name "Quercetin Capsules" from Alpha Science Laboratories, Toronto, Canada), equivalent to 500 mg of each, were then added to the gel mixture. Then, 1.5 ml of THC oil (containing 20 mg/ml of THC; purchased under the commercial name "Red Cannabis Oil" from Tweed Inc, Smiths Falls, Ontario, Canada) and 3.5 ml of CBD oil (containing 20 mg/ml of CBD; purchased under the commercial name "Yellow Cannabis Oil" from Tweed Inc, Smiths Falls, Ontario, Canada) were added. Finally, 3ml of beta-caryophyllene (containing 814.5 mg/ml of beta-caryophyllene; purchased from True Terpenes Inc, 2416 N Hayden Island Drive, Portland, Oregon, USA, 97217) and 1 ml of linalool (containing 852.9 mg/ml of linalool; purchased from True Terpenes Inc, 2416 N Hayden Island Drive, Portland, Oregon, USA, 97217) were added. The mixing container was then closed and shaken for 30 seconds. The mixing container was covered with dark tape in order to protect from the light. The prepared formulation contained a) 2.3 mg/ml of cannabidiol and 1.0 mg/ml of tetrahydrocannabinol; (b) 81.5 mg/ml of beta-caryophyllene and 28.4 mg/ml of linalool; and (c) 16.7 mg/ml of micronized diosmin and 16.7 mg/ml of micronized quercetin.

Example 2: A Case Report of Topical Formulation of Cannabinoids, Terpenes and Flavonoids in Wound Healing

This example demonstrated the efficacy of the topical formulation prepared in Example 1, which was topically instilled upon wound beds of chronic and recalcitrant wounds. The treatment was found to promote healing in chronically recalcitrant wounds.
A compounded mixture of cannabinoids, terpenoids and flavonoids was topically instilled onto the wound bed of an 85-year-old woman with a large chronic wound on her right leg since 2013. This patient also suffered from CHF, Atrial Fibrillation, and Osteoarthritis. The wound had clinical signs of idiopathic Pyoderma Gangrenosum, identified with histopathologic and immunofluorescent features. The patient refused traditional medicines such as Prednisone, Imuran, Cyclosporine, and Inflixamab.
The treatment started from November 1, 2017. The highly heterogeneous wound has seen rapid granulation and later epithelium growth inside the original necrotic area, and significant shrinking of the wound area over the course of 90 days. In addition to wound granulation, the patient also reported that the patient experienced reduced wound-related pain and exudation.
During the treatment of the patient, color photos of the wound were routinely taken at the patient's residence. As an alternative, the longest length and widest width of the wound were manually recorded.
During the period from November 1, 2017 to January 30, 2018 (total: 90 days), there were 35 visits to the patient (average frequency: 2.6 days/visit). 119 images (average: 3.4 images/visit) were analyzed. Multiple images were taken on each visit due to the large surface extent of the wound which could not fit into one camera field of view (FOV).
Wound boundaries were manually contoured on each image. The wound area was extracted from the contour using a polygon area mask defined by the contour points. This area is defined as the region of interest (ROI). A color space transformation from the Red-Green-Blue (RGB) space to the Hue-Saturation-Value (HSV) space was performed on the image. The HSV space (or other similar approaches) is commonly used to perform segmentation of different objects, including skin, due to its much higher contrast between semantically different objects. The hue space value is commonly represented by a non-dimensional value from 0 to 360 and wraps around. 0 and 360 represent red color while green is at 120 and blue is at 240. Since the color of interest is around the red wavelength, a different period in the hue space from -180 to 180 was taken. As a result, 0, 120 were still red and green respectively, but blue is shifted one period to (240-360)=-120, and most importantly, all pixels with red color would be next to each other for histogram analysis, allowing much more convenient binning. Finally, the range (-180,180) is normalized to (-0.5,0.5) for interoperability between different software platforms which may choose a different range for hue space (e.g. some may choose to let hue range from 0 to 255 to fit within a traditional 8-bit data structure). All pixels in the hue channel inside the ROI were counted on a histogram. Empirical thresholds were applied to classify the pixels inside the wound as "necrosis," "granulation," "epithelium," and "no label."

Assume the areas of necrosis, granulation, and epithelium would be rather contiguous, morphological operations were performed to improve accuracy of classification. First, an image erosion structuring element of appropriate size was applied so that small misclassified regions would be eliminated. Then, a dilation filter using the same structuring element is applied to recover the correct size of classified areas.

For visualization, a small dilation structuring element was applied to each area, followed by a subtraction of the area. This series of operation allows one to draw out the outer contour of the classified area. Areas of difference classifications were delineated by different contour colors.

Finally, the percentage of each type of pixel classification inside the wound was calculated to be interpreted as the percent of area inside the wound being the labeled type of tissue:

$$\% \ granulation = number \ of \ \text{"}granulation\text{"} \ pixels/total \ number \ of \ pixels \ in \ wound$$

$$\% \ epithelial = number \ of \ \text{"}epithelial\text{"} \ pixels/total \ number \ of \ pixels \ in \ wound$$

The entire pipeline of the analysis was implemented in the Python programming language using the OpenCV computer vision library.

In addition to the image analysis data, an upper-bound estimate of the wound area is provided by the measured widest length and longest length: *wound area≈longest length×widest width.*

Statistical analysis was performed in Python (version 3.6.2, SciPy version 0.19.1) and MATLAB (version R2016a).

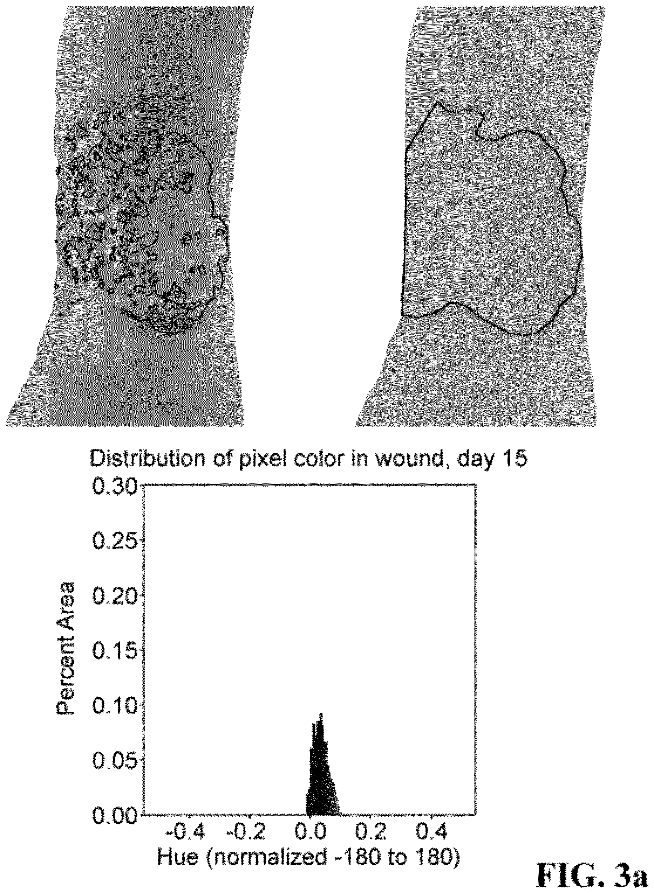

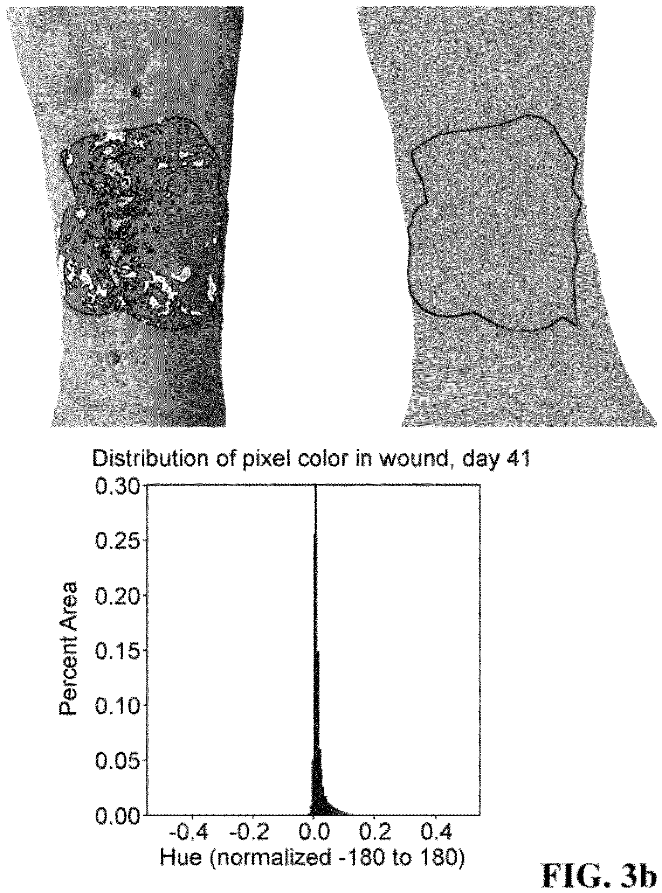

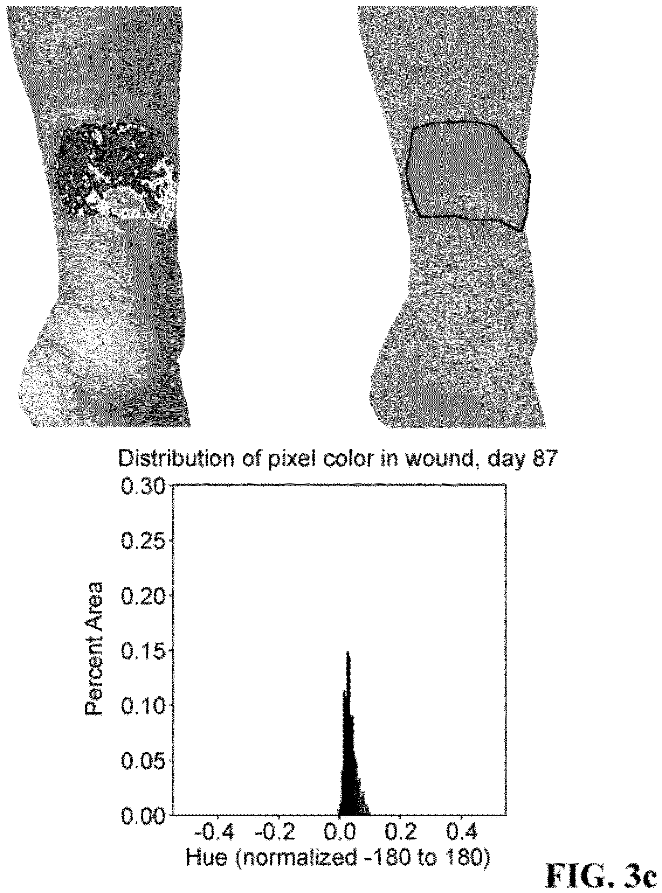

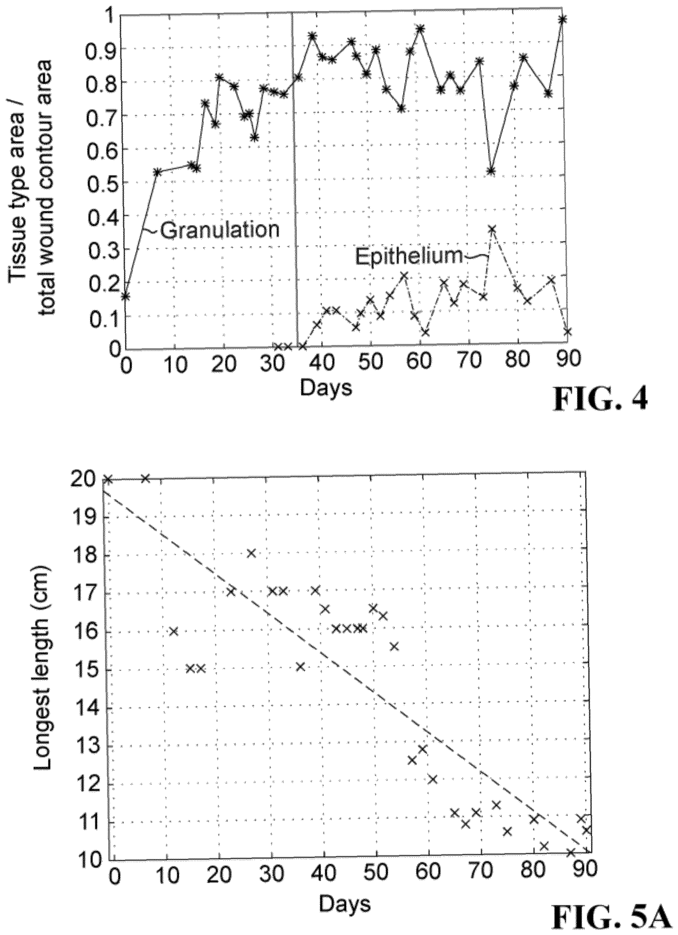

Representative images on days 15, 41, and 87 and their analysis are shown in FIGS. 3a-c. The upper left image of each figure was taken with the classified area contoured (dark grey: granulation, white: epithelial tissue). The upper right image of each figure shows the hue channel (the area enclosed within the solid line) calculated from each image. The bottom graph of each figure shows the hue value histogram inside the wound area. Note the peak becomes significantly higher and narrower from day 15 to 41 due to the increased percentage of granulation tissue, and the peak becomes slightly wider on day 87 due to the growing amount of epithelial tissue. The wound healing can be distinguished into two distinct phases: the first phase before day 30 when tissue started a rapid granulation before day 30, and the second phase after day 30 when granulation process has saturated at close to 100% and epithelium growth starts. This division can be seen clearly observed in the summarized statistics shown in FIG. 4.

As discussed in the Background section, the initial phase of granulation is characterized by the narrowing of the histogram peak and the increase in magnitude of the peak. In the second phase of healing where granulation saturates and epithelium starts to form, the histogram gradually grows into the yellow region with a decreased magnitude of peak, representing granulation tissue turning into epithelial tissue.

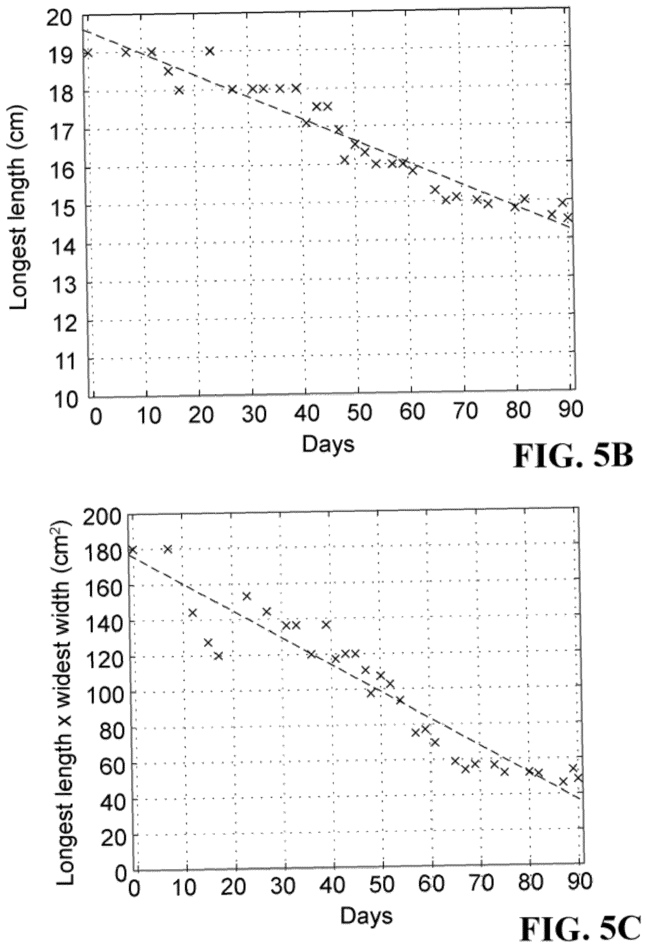

The trend of the wound size and proportion of granulation and epithelium tissue is shown in FIGS. 5a-c and FIG. 4 respectively. In FIGS. 5a-c, a linear regression line of all the data points represented by "X" is shown as a dotted line in each figure. The equation of the linear regression line of FIG. 5a is y=-0.106x+19.6 with a coefficient of determination ($R^2$) of 0.774; the equation of the linear regression line of FIG. 5b is y=-0.059x+9.6 with a coefficient of determination ($R^2$) of 0.930; and the equation of the linear regression line of FIG. 5c is y=-1.545x+175.8 with a coefficient of determination ($R^2$) of 0.903.

It is important to note that the two indices measure different quantities: while the total wound size is considered in FIGS. 5a-c, the composition inside the wound is considered in FIG. 4. This difference manifests in the strong linear decreasing trend in FIG. 5c where the product of length and width has $R^2$ = 0.903. In comparison, FIG. 4 shows rapid initial granulation and epithelium growth and saturation of the growth after a few days. This suggests that these two metrics are best to be observed in conjunction to fully understand the healing process of a wound.

The dramatic wound healing results observed in this case suggest the occurrence of a potentiated and synergistic effect between cannabinoids, terpenoids and flavonoids.

Example 3: A Series of N=1 Trials

A series of n=1 trials were initiated on a cohort of stalled recalcitrant wounds, composed of cases with an average chronicity of over 2 years, were treated with topical formulations of the present invention. The topical formulations were applied directly applied to wound beds, and in some cases, to wound beds and the periwound areas. All cases were previously afforded with all available Evidence Based Medicine treatments that conformed with local best practices and wound-bed preparation principles. All patients provided informed consent to be subjected to the experimental therapy.

The patient recruitment information is summarized below:

- "Complex Wounds" affecting "Complex Patients"
- 29 patients with 40 recalcitrant wounds
- Wound Diagnoses:

    o Venous Leg Ulcers
    o Arterial Leg Ulcers
    o Pyoderma Gangrenosum
    o Non-uremic Calciphylaxis
    o Diabetic foot ulcers
    o Pressure Ulcers
    o Antiphospholipid syndrome
    o Post-surgical wounds
    o Sickle Cell Disease

- Wound Duration: more than 6 months (Range 6 months to 11 Years)

    ∘ 27 patients with wounds involving cutaneous membranes
    ∘ 2 patients with wounds involving mucous membranes
    ∘ Gender:

        ▪ 23/29 female
        ▪ 6/29 male

    ∘ Age:

        ▪ Average = 71.6 years
        ▪ Range: 29 to 90

    ∘ Performance Status: (*Entirely healthy people score 100%*)

        ▪ Average = 68.3%
        ▪ Range: 10% to 90%

    ∘ Charlson Co-Morbidity Index: (*Entirely healthy people score 0*)

        ▪ Average = 4.79
        ▪ Range: 1 to 11.

The topical formulations used for treatment are identified in the table below.

| FORMULA | Aloe Vera Gel | Hyaluronic Acid Gel | Liposomal Base | Plutonic Lecithin Organogel | Normal Saline | Quercetin | Diosmin | Hersperidin | Kaempferol | Apigenin | CBD | THC | Beta-Carophyllene | Linalool |
|---|---|---|---|---|---|---|---|---|---|---|---|---|---|---|
| F1-2 | 10 ml | 10 ml | | | | 500 mg | | | | | 10 mg | | 3 ml | |
| F3 | 10 ml | 10 ml | | | | 500 mg | | | | | 7.5 mg | 5 mg | 3 ml | |
| F4 | 10 ml | 10 ml | | | | 500 mg | | | | | 30 mg | 20 mg | 3 ml | |
| F5 | 10 ml | 10 ml | | | | 500 mg | | | 500 mg | 500 mg | 10 mg | | 3 ml | |
| F6 | 20 ml | | | | | 1,000 mg | | | | | 50 mg | | 10 ml | |
| F7 | 10 ml | 10 ml | | | | 500 mg | 450 mg | 50 mg | | | 7.5 mg | 5 mg | 2 ml | 2 ml |
| F8 | 10 ml | 10 ml | | | | 500 mg | 450 mg | 50 mg | | | 55 mg | 10 mg | 3 ml | 3 ml |
| F9 | 10 ml | 10 ml | | | | 500 mg | 450 mg | 50 mg | 500 mg | 500 mg | 55 mg | 10 mg | 3 ml | 3 ml |
| F10 | 10 ml | 10 ml | | | | 500 mg | 450 mg | 50 mg | | | 60 mg | | | 3 ml |
| F11 | 10 ml | 10 ml | | | | 500 mg | 450 mg | 50 mg | | | 70 mg | 20 mg | 2 ml | |
| F12 | 10 ml | 10 ml | | | | 500 mg | 450 mg | 50 mg | | | 60 mg | | 3 ml | |
| F12B | 10 ml | 10 ml | | | | 500 mg | | | 500 mg | 500 mg | 60 mg | | 3 ml | 3 ml |
| F13 | 10 ml | 10 ml | | | | 500 mg | 450 mg | 50 mg | | | 60 mg | 30 mg | 3 ml | 3 ml |

| FORMULA | Aloe Vera Gel | Hyaluronic Acid Gel | Liposomal Base | Plutonic Lecithin Organogel | Normal Saline | Quercetin | Diosmin | Hersperidin | Kaempferol | Apigenin | CBD | THC | Beta-Caro-phyllene | Linalool |
|---|---|---|---|---|---|---|---|---|---|---|---|---|---|---|
| F14 | | | 10 ml | | | 500 mg | 450 mg | 50 mg | | | 60 mg | | 3 ml | |
| F15 | | | | 10 ml | | 500 mg | 450 mg | 50 mg | | | 60 mg | | 3 ml | |
| F16 | 20 ml | | | | | 1,000 mg | | | | | 60 mg | | 3 ml | |
| F17 | | | 10 ml | | | 500 mg | 450 mg | 50 mg | | | 50 mg | 50 mg | 5 ml | |
| F18 | | | 10 ml | | | 500 mg | 450 mg | 50 mg | | | 100 mg | | 10 ml | |
| F19 | | | 10 ml | | | 500 mg | 450 mg | 50 mg | | | 50 mg | 25 mg | 5 ml | 3 ml |
| F20 | | | | | 100 ml | 1,000 mg | 450 mg | 50 mg | | | 100 mg | 50 mg | 10 ml | 5 ml |

EP 4 252 778 A2

Among the 29 patients treated, complete wound closure was observed in 20 patients, progressive wound closure was observed in 7 patients, and no significant wound closure was observed in 2 patients (one with severe diabetic charcot foot and osteomyelitis and the other with severe non-uremic calciphylaxis). All patients experienced clinically relevant pain relief within 5-7 days. Reduced utilization of opioid analgesics and reduced utilization of systemic antibiotics were also observed, while no systemic or local adverse reactions were observed. No patient underwent amputation.

Example 4: A Case Report of Medical Cannabis in the Palliation of Malignant Wounds

A 44-year-old man with an exophytic (fungating) wound involving his right cheek area was diagnosed with a squamous cell cancer of his right buccal cavity three years earlier. He had the tumor surgically resected, followed by external beam radiotherapy and chemotherapy. Despite this appropriate cancer treatment, he developed a buccal recurrence that eventually eroded through his cheek, creating an oral cutaneous fistula and associated exophytic lesion. Over the two-year period before treatments with medical cannabis (MC), he had elected to forego further conventional oncologic therapies in favor of mostly naturopathic treatments. Despite using high-dose hydromorphone, pregabalin, and dexamethasone, he continued to experience continuous (background) generalized right hemifacial pain along with volitional incident pain (wound-related procedural pain) occurring with wound dressing changes. He rated his average daily pain score as 9 of 10. In addition, he also reported having side effects from his analgesics, such as constipation and drowsiness. He also reported suffering severe aesthetic distress from his facial disfigurement along with right-sided trismus, depression, insomnia, nausea, and anorexia.

At the beginning, he was offered a trial of vaporized MC (ARGYLE™; THC 7.25% + CBD 8.21%) from TWEED, Inc. delivered through a certified Volcano™ vaporizer unit. The particular strain was strategically chosen to maximize the analgesic potential of both THC and CBD while mitigating against the sedation and psychotomimetic side effects commonly experienced with high-dose THC strains.

On his second clinic visit, he reported significant reductions in both baseline and volitional incident pain. He indicated that he used 0.5-1.0 g of dried cannabis per day and vaporized every two to four hours and 15 minutes before his daily wound dressing change. His pain relief was so significant that he was able to discontinue pregabalin and dexamethasone while reducing hydromorphone to approximately 25% of his pre-MC dosage. He also reported experiencing less trismus and nausea, along with improved appetite, sleep, and effect. Importantly, he reported no negative effects from the MC. Furthermore, his overall performance status and symptom control was good enough to allow him to be working modified hours as a health care professional.

During his third and fourth clinic visits, his malignant wound was observed to have increased in size, yet his performance status only marginally declined, and his average daily pain scores remained within tolerable limits, while needing only small increases in daily opioid utilization. Unfortunately, his trismus and oral cutaneous fistula rendered the continued use of vaporized MC technically difficult.

Because the patient had experienced such positive outcomes with MC therapy, he was eager to continue it through an alternate delivery system. Thus, he was offered a trial of topical MC compounded in nongenetically modified organic sunflower oil (ARGYLE THC 5.24% + CBD 8.02% from TWEED, Inc.). He was instructed to apply, and digitally spread, 1-2 cc of the MC oil to the entire malignant wound, both externally and intrabuccal. He also was advised to swish any residual oil throughout his oral cavity and swallow any residual.

On his fifth clinic visit, he reported having consistently used the topical MC four times daily. He stated that pain relief commenced with 10-15 minutes after application and lasted for up to two hours after application. He did not report any negative experiences from the use of topical MC. Between his fourth and fifth clinic visits, his condition began to globally deteriorate, and he required a doubling in his daily opioid utilization. Interestingly, the size of his malignant wound decreased by about 5% over the four week interval.

Four weeks after his last clinic visit, he was admitted to an acute general hospital with hypovolemia. As a result, he was lost to follow-up and ceased to use MC on his admission. He expired three weeks later.

His clinical course over five months of treatments with MC is summarized in Table 1.

*Table 1*

**Clinical Data**

| Date | Tumor Size (cm$^2$) | Average Daily Pain Score (0-10) | Analgesics | MC Therapy | PPSv2 (%) |
|---|---|---|---|---|---|
| November 12, 2015 | 8.75 | 9 | Hydromorphone 30 mg/day<br>Pregabalin 150 mg/day<br>Decadron 4 mg/day | Vaporized | 90 |

(continued)

**Clinical Data**

| Date | Tumor Size (cm$^2$) | Average Daily Pain Score (0-10) | Analgesics | MC Therapy | PPSv2 (%) |
|---|---|---|---|---|---|
| December 10, 2015 | 12.33 | 3 | Hydromorphone 8 mg/day | Vaporized | 90 |
| January 21, 2016 | 26.44 | 3 | Hydromorphone A mg/day | Vaporized | 80 |
| March 17, 2016 | 44.16 | 4 | Hydromorphone 10 mg/day | Topical Oil | 70 |
| April 21, 2016 | 41.90 | 4 | Hydromorphone 20 mg/day | Topical Oil | 60 |

MC = medical cannabis; PPSv2 = Palliative Performance Scale, version 2.

This case report demonstrates the potential for MC to provide effective pain and symptom management in the setting of malignant wounds. The rapid onset of analgesia after topical placement suggests that the effects were mediated through absorption of the THC and CBD cannabinoids that subsequently interacted with peripheral nociceptors, immune cells, and cancer cells. The post application analgesia may be because of the gastrointestinal absorption of ingested residual MC oil.

Example 5: 3 Case Reports of Topical Medical Cannabis in the Treatment of Patients with Pyoderma Gangrenosum

Pyoderma gangrenosum (PG) is a rare inflammatory neutrophilic skin disease. Although 50-70% of cases occur in the setting of inflammatory arthritis, inflammatory bowel disease, hematologic diseases, and solid neoplasms, the remainder are idiopathic. Classically, it presents as cutaneous ulcerations that most commonly occur on the lower extremities. PG represents a significant challenge from both diagnostic and therapeutic perspectives. PG is frequently misdiagnosed as cellulitis, venous leg ulcers, and arterial ulcers. Pain is a universal symptom of PG and most patients suffer high levels of pain that is often refractory to high-dose systemically administered opioid analgesics. Because the lesions of PG tend to be chronic and relapsing, they have the potential to substantially compromise quality of life over a protracted period.

*Methods*

Before the initiation of topical medical cannabis (TMC), all patients underwent a complete medical workup and providing informed consent for the use of this experimental treatment. All patients were also subjected to wound biopsies for histopathology and immunofluorescence studies to rule out other pathologies. For all three cases, patient reported average daily pain scores, based on an 11-point numeric rating scale (0-10), and average daily opioid use (morphine sulfate equivalents in mg/day) were assessed before and after initiating treatment with TMC. Using a paired t-test, the mean pre-TMC average daily pain score was compared with the mean post-TMC value for all three cases. The percent decrease in average daily pain score after the initiation of TMC was also determined for each case. For average daily opioid dosage, a paired t-test was used to compare the mean pre-TMC morphine sulfate equivalents (MSE) used to the mean post-TMC values for cases 1 and 2 only. In Case 3, the mean MSEs used was nil both before and after initiating treatment with TMC, precluding comparison with a paired t-test. For all hypothesis testing, a P-value <0.05 was considered significant and a decrease in average pain score greater or equal to 30% was accepted to be clinically significant. All statistical analyses were carried out using GraphPad QuickCalcs Software (GraphPad Software Inc., La Jolla, CA).

*Case 1*

A 50-year-old woman presented with a painful left medial leg ulcer of at least 12months' duration. This PG was superimposed on an area of lipodermatosclerosis resulting from a post-phlebitic syndrome in the setting of Factor V Leiden deficiency. She was initially treated with systemic corticosteroids, intralesional corticosteroids, opioid analgesics, and inelastic compression systems. In view of her continued high levels of pain, she agreed to a trial of topical MC oil (ARGYLE™ THC 5mg/mL + CBD 6 mg/mL) from TWEED Inc (Ontario, Canada). One milliliter of TMC was applied to wound bed daily followed by application of inelastic compression bandaging. The use of the multilayered inelastic compression system precluded the use of TMC for breakthrough pain in this case. After the initiation of TMC, she did not require further corticosteroids.

*Case 2*

A 76-year-old man, with no concomitant illnesses, presented with the first-ever occurrence of a painful right lateral ankle ulcer. He was prescribed opioid analgesics and systemic corticosteroids both before and after the initiation of TMC. Before initiating TMC, he was also administered intralesional corticosteroids. He continued to experience high levels of pain and, thus, he agreed to a trial of MC oil (Bedrolite™ THC 7 mg/mL + CBD 9 mg/mL) from Bedrocan Inc. He applied 0.5-1.0 mL of MC oil to the wound bed two times per day plus one to three times daily for breakthrough pain. The wound was dressed with nonadherent dressings.

*Case 3*

A 60-year-old woman with systemic lupus erythematosus presented with a recurrent painful right lateral leg ulcer. She was prescribed systemic corticosteroids both before and after the initiation of TMC. She had a history of side effects with opioid analgesics and, thus, refused to use them. She used acetaminophen 325-650 mg every 6 hours as needed for pain. Given her high levels of pain, she agreed to a trial of MC oil (Bedrolite™ THC 7 mg/mL + CBD 9 mg/mL) from Bedrocan Inc. She applied 0.5-1.0 mL of MC oil to the wound bed two times per day plus one to three times daily for breakthrough pain. The wound was dressed with nonadherent dressings.

*Results*

The data in Tables 2 and 3, collected prospectively, reflect clinical observations over a total of 17, 21, and 12 weeks for cases 1-3 pre-TMC, respectively, and over 33, 9, and 21 weeks for cases 1-3 post-TMC, respectively. Each of the three patients reported consistently experiencing the onset of analgesia within three to five minutes of each application. After the initiation of treatment with TMC, there was a statistically significant ($P < 0.05$) decrease in the average daily pain score in cases 1 and 2 (Table 2). In addition, all cases demonstrated "clinically significant" pain reductions of greater than 30% which is the generally accepted threshold quoted in international pain research (Younger et al., Curr. Pain Headache Rep. 2009;13:39-43). In Case 1, the mean pain score decreased from 8.25 to 2.76, a 66.5% decrease that is both clinically and statistically significant (P = 0.0007). For Case 2, the pre-TMC mean pain score was 8.75, which decreased by 73.4% to 2.33, a clinically and statistically significant (P = 0.0006) change. Finally, for Case 3, the mean pain score decreased from 4.29 to 1.50, a 65% change that was clinically significant but did not quite reach the threshold for statistical significance (P = 0.0720). The average daily opioid dose in cases 1 and 2, measured as MSE (mg), decreased in a statistically significant manner after starting the application of TMC (Table 3). For Case 1, the mean MSE decreased from 26.00 to 0.24 mg, a statistically difference (*P* = 0.0013). In Case 2, mean MSE decreased from 27.33 mg to 12.50, a decrease that was also statistically significant (*P* = 0.0001).

This case series demonstrates the potential for TMC to provide effective analgesia that was opioid sparing in the setting of PG. The rapid onset of analgesia after topical application suggests that the effects were mediated through absorption of the cannabinoids THC and CBD that subsequently interacted with cannabinoid receptors expressed on peripheral nociceptors and immune cells.

*Table 2*

**Comparison of Mean Daily Pain Scores Before and After Initiating Treatment With TMC**

| Case | Mean Daily Pain Score Pre-TMC ± SD (*n*) | Mean Daily Pain Score Post-TMC ± SD (*n*) | *P*-Value | Percent Change (%) |
|---|---|---|---|---|
| 1 | 8.25 ± .50 (4) | 2.76 ± 1.34 (25) | 0.0007 | 66.5 |
| 2 | 8.75 ± .46 (8) | 2.33 ± 1.97 (6) | 0.0006 | 73.4 |
| 3 | 4.29 ± .95 (4) | 1.50 ± 1.60 (7) | 0.0720 | 65.0 |

TMC = topical medical cannabis.

*Table 3*

**Comparison of Mean MSE Before and After Initiating Treatment With TMC**

| Case | Mean MSE (mg/day) Pre-TMC ± SD (*n*) | Mean MSE (mg/day) Post-TMC ± SD (*n*) | *P*-Value |
|---|---|---|---|
| 1 | 26.00 ± 5.16 (4) | 0.24 ± .88 (25) | 0.0013 |
| 2 | 27.33 ± 2.18 (8) | 12.50 ± 1.23 (6) | 0.0001 |

(continued)

**Comparison of Mean MSE Before and After Initiating Treatment With TMC**

| Case | Mean MSE (mg/day) Pre-TMC ± SD (*n*) | Mean MSE (mg/day) Post-TMC ± SD (*n*) | *P*-Value |
|---|---|---|---|
| 3 | 0 (4) | 0 (7) | n/a |
| MSE = morphine sulfate equivalents; TMC = topical medical cannabis; n/a = not applicable. | | | |

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The compounds described herein may contain one or more chiral centers and/or double bonds and therefore, may exist as stereoisomers, such as double-bond isomers (i.e., geometric isomers such as E and Z), enantiomers or diastereomers. The present disclosure includes each of the isolated stereoisomeric forms (such as the enantiomerically pure isomers, the E and Z isomers, and other alternatives for stereoisomers) as well as mixtures of stereoisomers in varying degrees of chiral purity or percentage of E and Z, including racemic mixtures, mixtures of diastereomers, and mixtures of E and Z isomers.

Accordingly, the compounds described herein encompass all possible enantiomers and stereoisomers thereof including the stereoisomerically pure form (e.g., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The present disclosure includes each of the isolated stereoisomeric forms as well as mixtures of stereoisomers in varying degrees of chiral purity, including racemic mixtures. It also encompasses the various diastereomers.

When the chemical name does not specify the isomeric form of the compound, it denotes any one of the possible isomeric forms or mixtures of those isomeric forms of the compound. The compounds may also exist in several tautomeric forms. Accordingly, the compounds depicted herein encompass all possible tautomeric forms thereof.

The term "tautomer" is generally understood to refer to isomers that change into one another with great ease so that they can exist together in equilibrium; the equilibrium may strongly favor one of the tautomers, depending on stability considerations. For example, ketone and enol are two tautomeric forms of one compound.

It must be noted that as used in this specification and the appended claims, the singular forms "a," "an," and "the" include plural reference unless the context clearly dictates otherwise. Unless defined otherwise all technical and scientific terms used herein have the same meaning as commonly understood to one of ordinary skill in the art to which this invention belongs.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to encompass the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items.

As used herein, whether in the specification or the appended claims, the transitional terms "comprising", "including", "having", "containing", "involving", and the like are to be understood as being inclusive or open-ended (i.e., to mean including but not limited to), and they do not exclude unrecited elements, materials or method steps. Only the transitional phrases "consisting of" and "consisting essentially of", respectively, are closed or semi-closed transitional phrases with respect to claims and exemplary embodiment paragraphs herein. The transitional phrase "consisting of" excludes any element, step, or ingredient which is not specifically recited. The transitional phrase "consisting essentially of" limits the scope to the specified elements, materials or steps and to those that do not materially affect the basic characteristic(s) of the invention disclosed and/or claimed herein.

**Claims**

1. A topical instillate comprising:

   (a) 0.1 mg/ml to 40 mg/ml of one or more cannabinoids;
   (b) 25 mg/ml to 1000 mg/ml of one or more terpenes;
   (c) 10 mg/ml to 500 mg/ml of one or more flavonoids; and
   (d) a liquid carrier selected for instillation of the instillate onto an integumentary wound.

2. The topical instillate of claim 1, wherein the one or more cannabinoids comprise at least one of cannabidiol and tetrahydrocannabinol.

3. The topical instillate of claim 1 or claim 2, wherein the one or more terpenes comprise beta-caryophyllene, and the concentration of beta-caryophyllene is 50 mg/ml to 500 mg/ml.

4. The topical instillate of any one of claims 1 to 3, wherein the one or more flavonoids comprise at least one of diosmin, quercetin, and hesperidin.

5. The topical instillate of any one of claims 1 to 4, wherein the one or more terpenes further comprise linalool, and the concentration of linalool is 10 mg/ml to 150 mg/ml.

6. The topical instillate of any one of claims 1 to 5, wherein the liquid carrier comprises an aloe vera gel and a hyaluronic acid gel.

7. A wound dressing comprising a contact layer, and the topical instillate of any one of claims 1 to 6 integrated with the contact layer.

8. A topical formulation for use in the treatment of an integumentary wound of a subject, wherein the topical formulation comprises one or more cannabinoids; one or more terpenes; and one or more flavonoids.

9. The topical formulation for use of claim 8, wherein the topical formulation is used for instillation onto the integumentary wound.

10. The topical formulation for use of claim 8 or 9, wherein the integumentary wound is acute, or is chronic, stalled, recalcitrant, or a combination thereof

11. The topical formulation for use of any one of claims 8 to 10, wherein the topical formulation comprises the topical instillate of any one of claims 1 to 6.

12. The topical formulation for use of any one of claims 8 to 11, wherein the topical formulation is further used for instillation onto a periwound area around the integumentary wound.

13. The topical formulation for use of any one of claims 8 to 12, wherein the integumentary wound is caused by a skin disease or condition, wherein the skin disease or condition is Skin Cancer (Primary Neoplasms & Metastatic Neoplasms), Integumentary Ulcers and Erosions caused by microbes (e.g., a bacterium, fungus, virus, and mycobacterium), Pyoderma Gangrenosum, Leukocytoclastic Vasculitis, Cryoglobulinemia, Cryofibrinogenemia, Antiphospholipid Syndrome, Sickle Cell Disease, Lichen Simplex Chronicus, Bowen's Disease, Martorell's Ulcer, Calciphylaxis, Allergic Dermatitis, Psoriasis, Epidermolysis Bullosa, Pemphigus, Bullous Pemphigoid, Behcet's Disease, Rheumatoid arthritis, Systemic Lupus Erythematosis, Scleroderma, Wegeners Granulomatosis, or Hidradenitis Suppurativa.

14. The topical formulation for use of any one of claims 8 to 13, which stimulates granulation tissue growth.

15. A kit comprising (i) a container containing the topical instillate of any one of claims 1 to 6, and instructions for instilling the topical instillate onto an integumentary wound; or (ii) a plurality of containers containing materials for forming the topical instillate, and instructions for preparing the topical instillate from the materials in the containers and for instilling the topical instillate onto the integumentary wound.

**FIG. 1**
**(Prior Art)**

FIG. 2a

FIG. 2b

FIG. 2c

FIG. 2d

FIG. 2e

FIG. 2f

FIG. 2g

Distribution of pixel color in wound, day 15

FIG. 3a

Distribution of pixel color in wound, day 41

FIG. 3b

Distribution of pixel color in wound, day 87

FIG. 3c

FIG. 4

FIG. 5A

FIG. 5B

FIG. 5C

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017190249 A
- US 20140271940 A
- US 20160250270 A

### Non-patent literature cited in the description

- **BIELEFELD et al.** *Cell. Mol. Life Sci,* 2013, vol. 70, 2059-2081
- **OLIVEIRA GONZALEZ et al.** *An Bras Dermatol,* 2016, vol. 91 (5), 614-20
- **DHIVYA S et al.** *Biomedicine (Taipei),* December 2015, vol. 5 (4), 24-28
- **SIBBALD RG et al.** *Journal of Cutaneous Medicine and Surgery,* 2013, vol. 17 (4), S12-S22
- **YOUNGER et al.** *Curr. Pain Headache Rep,* 2009, vol. 13, 39-43